(19) Europäisches Patentamt
European Patent Office
Office européen des brevets

(11) EP 2 607 361 A1

# (12) EUROPEAN PATENT APPLICATION

(43) Date of publication:
**26.06.2013 Bulletin 2013/26**

(51) Int Cl.:
***C07D 331/02*** *(2006.01)* ***A61P 31/04*** *(2006.01)*

(21) Application number: **11194584.6**

(22) Date of filing: **20.12.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Ludwig-Maximilians-Universität München**
**80539 München (DE)**

(72) Inventors:
- **Sieber, Stephan**
  **86919 Utting am Ammersee (DE)**
- **Pitschneider, Maximilian**
  **81475 Munich (DE)**

(74) Representative: **Vossius & Partner**
**Siebertstrasse 4**
**81675 München (DE)**

## (54) Thiirane and michael acceptor compounds and their medical use

(57) The present invention relates to thiirane or Michael acceptor compounds, including the compounds of formula (I) as described and defined herein, and pharmaceutical compositions comprising these compounds, as well as their medical use, particularly their use in the treatment or prevention of a bacterial infection, including, e.g., an infection with multidrug-resistant *Staphylococcus aureus.*

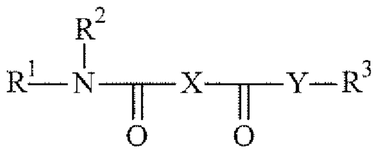

(I)

EP 2 607 361 A1

## Description

The present invention relates to thiirane or Michael acceptor compounds, including the compounds of formula (I) as described and defined herein, and pharmaceutical compositions comprising these compounds, as well as their medical use, particularly their use in the treatment or prevention of a bacterial infection, including, e.g., an infection with multidrug-resistant *Staphylococcus aureus.*

With the development of antibiotic resistant bacterial strains, such as multidrug-resistant *Staphylococcus aureus* (MRSA), infectious diseases have become again a life threatening problem (Taubes, G., Science, 2008, 321(5887): 356-361). One of the reasons for this dilemma is the limited number and breadth of current therapeutic targets for which several resistance strategies have evolved over time. Today 500,000 people are infected with MRSA every year and 100,000 die due to the lack of treatment options (AHRQ, C.f.D., Organization, and Markets, Healthcare Cost and Utilization Project, Nationwide Inpatient Sample, 1993-2005). Since the majority of current antibiotics addresses only the classical repertoire of cellular functions such as DNA replication, protein and cell wall biosynthesis, it is an urgent goal to identify novel, customized targets in pathogenic and resistant bacteria for functional characterization and subsequent drug development.

The field of anti-bacterial research is confronted with several challenges that include the development and application of new and innovative technologies which are potent enough to identify novel antibacterial drug targets that are essential for resistance, viability or pathogenesis and their corresponding inhibitors. In this regard nature provides a rich source of so far unexplored bioactive compounds that comprise a huge diversity of pharmacological activities (Clardy, J. et al., Nat Biotechnol, 2006, 24(12): 1541-1550; Clardy et al., Nature, 2004, 432(7019): 829-837). The anti-bacterial targets that are addressed by natural products are as diverse as their structures. Some compounds interfere with essential enzymes that are involved in primary metabolism, others utilize the cell wall as an essential point for attack. In order to selectively address this diversity of targets, natural products usually comprise very fine tuned structural compositions that range from simple to highly complex, probably depending on the desired target selectivity. It is an intriguing feature of many natural compounds that some privileged motifs are repetitive and indispensible for their bioactivity. Among these structural entities are small electrophilic reactive groups such as beta-lactams, beta-lactones, Michael acceptor systems and epoxides. Epoxides, e.g., cerulenin (Omura S., Bacteriol Rev, 1976, 40(3): 681-697; Young K. et al., Antimicrob Agents Chemother, 2006, 50(2): 519-526; US 5,539,132) or epoxides derived from the natural product E-64, have been extensively studied for bioactivity and in order to identify their functional targets in the past. Aziridine alkaloids belong to a rare and somewhat neglected class of natural products that exhibit potent antibiotic and anticancer activities but their mechanism of action is unknown. Although there has been only a small number of naturally occurring thiiranes identified so far, the corresponding bioactivities are quite potent and diverse, ranging from anticancer to immunosuppressant and antibacterial activities. For example, epitiostanol, an anabolic steroid, is an effective anti-tumor drug acting against breast cancer (Chew, W.a.H.D., Sulfur reports, 1993, 15: 1-39). Oxirane, thiirane and aziridine scaffolds represent core motifs in many natural products that may sometimes exhibit their bioactivity by a covalent modification of essential active site residues via a nucleophilic ring opening reaction.

There is an urgent demand for new approaches to discover targets and corresponding lead compounds. In the context of the present invention, it has surprisingly been found that the heteroatom of certain three-membered heterocycles is crucial for proteome reactivity and enzyme selectivity and, in particular, a thiirane based compound was identified as a dual inhibitor of FabF and FabH - which are two essential metabolic enzymes in pathogenic bacteria such as *Staphylococcus* and *Listeria* (Wang, J. et al., Proc Natl Acad Sci USA, 2007, 104(18): 7612-7616; Wang, J. et al., Nature, 2006, 441(7091): 358-361) - having potent antibiotic activity. Structural analogues of this compound with an oxirane or aziridine reactive group have been found to lack the advantageously high selectivity and antibiotic potency observed for the thiirane compound according to the invention, emphasizing the importance of fine tuned electrophilicity in natural products. Accordingly, the heteroatom identity of such three-membered heterocyclic compounds significantly contributes to the probe binding preferences for certain protein targets, emphasizing that not only affinity which depends on the attached ligands but also an optimal reactivity is important to discriminate enzyme active sites.

The present inventors have identified compounds that target more than one key enzyme in pathogenic bacteria, e.g., by the dual inhibition of the essential enzymes FabH and FabF, which is particularly valuable as resistance development is one of the most urgent problems in current anti-infective research efforts. The thiirane or Michael acceptor compounds of the present invention have further been demonstrated to be potent antibiotic agents, indicating their usefulness in the therapy of bacterial infections, including infections with multidrug-resistant pathogenic bacteria such as multidrug-resistant *S. aureus.*

The present invention thus solves the problem of providing improved therapeutic agents which are effective, *inter alia,* in the treatment or prevention of bacterial infections.

Accordingly, the present invention provides a compound of the following formula (I)

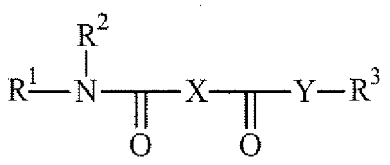

(I)

or a pharmaceutically acceptable salt, solvate or prodrug thereof. X is thiiran-2,3-diyl (i.e.,

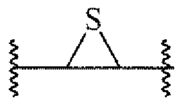 ) or ethen-1,2-diyl (i.e., 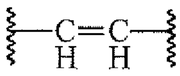).

Preferably, X is thiiran-2,3-diyl. It is furthermore preferred that the two moieties attached to X (i.e., the moiety $-CO-N(R^1)R^2$ and the moiety $-CO-Y-R^3$) are in trans-configuration with respect to the group X.

Y is -O- or -S-. Preferably, Y is -O-.

$R^1$ is selected from $C_{1-8}$ alkyl, $C_{2-8}$ alkenyl, or $C_{2-8}$ alkynyl, wherein said alkyl, said alkenyl or said alkynyl may be interrupted by one or more groups (e.g., one, two, three, or four groups) independently selected from -O-, -S-, -N(H)-, $-N(C_{1-5}$ alkyl)-, -C(=O)-, -C(=O)-N(H)-, $-C(=O)-N(C_{1-5}$ alkyl)-, -N(H)-C(=O)-, or $-N(C_{1-5}$ alkyl)-C(=O)-, and further wherein said alkyl, said alkenyl or said alkynyl may be substituted with one or more groups (e.g., one, two, or three groups) independently selected from halogen, $-CF_3$, -CN, -OH, $-O(C_{1-5}$ alkyl), -SH, $-S(C_{1-5}$ alkyl), $-NH_2$, $-NH(C_{1-5}$ alkyl), $-N(C_{1-5}$ alkyl)($C_{1-5}$ alkyl), $-CO-(C_{1-5}$ alkyl), $-CO-NH_2$, $-CO-NH-(C_{1-5}$ alkyl), $-CO-N(C_{1-5}$ alkyl)($C_{1-5}$ alkyl), $-NH-CO-(C_{1-5}$ alkyl), $-N(C_{1-5}$ alkyl)-CO-($C_{1-5}$ alkyl), aryl (e.g., phenyl), or heteroaryl (e.g., pyridinyl).

The one or more interrupting groups (i.e., the groups by which the above alkyl, alkenyl or alkynyl may be interrupted) are preferably selected independently from -O- or -S-, and are more preferably each -O-.

The one or more substituting groups (i.e., the groups with which the above alkyl, alkenyl or alkynyl may be substituted) are preferably selected independently from halogen, $-CF_3$, -CN, -OH, $-O(C_{1-5}$ alkyl), -SH, $-S(C_{1-5}$ alkyl), $-NH_2$, $-NH(C_{1-5}$ alkyl), or $-N(C_{1-5}$ alkyl)($C_{1-5}$ alkyl). More preferably, the one or more substituting groups are independently selected from -OH or $-O(C_{1-5}$ alkyl).

Preferably, $R^1$ is selected from $C_{1-8}$ alkyl, $C_{2-8}$ alkenyl, or $C_{2-8}$ alkynyl, wherein said alkyl, said alkenyl or said alkynyl may be interrupted by one or more (e.g., one, two, three, or four) groups independently selected from -O- or -S-, and further wherein said alkyl, said alkenyl or said alkynyl may be substituted with one or more (e.g., one, two, or three) groups independently selected from halogen, $-CF_3$, -CN, -OH, $-O(C_{1-5}$ alkyl), -SH, $-S(C_{1-5}$ alkyl), $-NH_2$, $-NH(C_{1-5}$ alkyl), or $-N(C_{1-5}$ alkyl)($C_{1-5}$ alkyl). Accordingly, $R^1$ may be, for example, $-(CH_2-CH_2-O)_p-H$ or $-(CH_2-CH_2-O)_q-CH_2-CH_3$, wherein p is 1, 2, 3 or 4, and q is 1, 2 or 3. More preferably, $R^1$ is selected from $C_{1-8}$ alkyl, $C_{2-8}$ alkenyl, or $C_{2-8}$ alkynyl, wherein said alkyl, said alkenyl or said alkynyl may be interrupted by one or more (e.g., one, two, three, or four) -O- groups, and further wherein said alkyl, said alkenyl or said alkynyl may be substituted with one or more (e.g., one, two, or three) groups independently selected from halogen, $-CF_3$, -CN, -OH, $-O(C_{1-5}$ alkyl), -SH, $-S(C_{1-5}$ alkyl), $-NH_2$, $-NH(C_{1-5}$ alkyl), or $-N(C_{1-5}$ alkyl)($C_{1-5}$ alkyl). Even more preferably, $R^1$ is selected from $C_{1-6}$ alkyl, $C_{2-6}$ alkenyl, or $C_{2-6}$ alkynyl, wherein said alkyl, said alkenyl or said alkynyl may be interrupted by one or more (e.g., one, two, three, or four) -O- groups, and further wherein said alkyl, said alkenyl or said alkynyl may be substituted with one or more (e.g., one, two, or three) groups independently selected from -OH, or $-O(C_{1-5}$ alkyl). Yet even more preferably, $R^1$ is selected from $C_{1-4}$ alkyl (e.g., $-CH_2-CH_2-CH_3$), $C_{2-4}$ alkenyl, or $C_{2-4}$ alkynyl (e.g., $-CH_2-C\equiv CH$). It is particularly preferred that $R^1$ is $-CH_2-C\equiv CH$.

$R^2$ is selected from H or $C_{1-5}$ alkyl. Preferably, $R^2$ is H, methyl or ethyl. More preferably, $R^2$ is H.

$R^3$ is selected from $C_{1-14}$ alkyl, $C_{2-14}$ alkenyl, or $C_{2-14}$ alkynyl, wherein said alkyl, said alkenyl or said alkynyl may be interrupted by one or more groups (e.g., one, two, or three groups) independently selected from -O-, -S-, -N(H)-, $-N(C_{1-5}$ alkyl)-, -C(=O)-, -C(=O)-N(H)-, $-C(=O)-N(C_{1-5}$ alkyl)-, -N(H)-C(=O)-, or $-N(C_{1-5}$ alkyl)-C(=O)-, and further wherein said alkyl, said alkenyl or said alkynyl may be substituted with one or more groups (e.g., one, two, or three groups) independently selected from halogen, $-CF_3$, -CN, -OH, $-O(C_{1-5}$ alkyl), -SH, $-S(C_{1-5}$ alkyl), $-NH_2$, $-NH(C_{1-5}$ alkyl), $-N(C_{1-5}$ alkyl)($C_{1-5}$ alkyl), $-CO-(C_{1-5}$ alkyl), $-CO-NH_2$, $-CO-NH-(C_{1-5}$ alkyl), $-CO-N(C_{1-5}$ alkyl)($C_{1-5}$ alkyl), $-NH-CO-(C_{1-5}$ alkyl), or $-N(C_{1-5}$ alkyl)-CO-($C_{1-5}$ alkyl), aryl (e.g., phenyl), or heteroaryl (e.g., pyridinyl).

The one or more interrupting groups (i.e., the groups by which the above alkyl, alkenyl or alkynyl may be interrupted) are preferably selected independently from -O- or -S-, and are more preferably each -O-.

The one or more substituting groups (i.e., the groups with which the above alkyl, alkenyl or alkynyl may be

substituted) are preferably selected independently from halogen, $-CF_3$, -CN, -OH, $-O(C_{1-5}$ alkyl), -SH, $-S(C_{1-5}$ alkyl), $-NH_2$, $-NH(C_{1-5}$ alkyl), $-N(C_{1-5}$ alkyl)$(C_{1-5}$ alkyl), aryl (e.g., phenyl), or heteroaryl (e.g., pyridinyl). More preferably, the one or more substituting groups are independently selected from halogen, $-CF_3$, -CN, -OH, $-O(C_{1-5}$ alkyl), -SH, $-S(C_{1-5}$ alkyl), $-NH_2$, $-NH(C_{1-5}$ alkyl), $-N(C_{1-5}$ alkyl)$(C_{1-5}$ alkyl), or phenyl.

Preferably, $R^3$ is selected from $C_{1-12}$ alkyl, $C_{2-12}$ alkenyl, or $C_{2-12}$ alkynyl, wherein said alkyl, said alkenyl or said alkynyl may be substituted with one or two groups independently selected from halogen, $-CF_3$, -CN, -OH, $-O(C_{1-5}$ alkyl), -SH, $-S(C_{1-5}$ alkyl), $-NH_2$, $-NH(C_{1-5}$ alkyl), $-N(C_{1-5}$ alkyl)$(C_{1-5}$ alkyl), aryl (e.g., phenyl), or heteroaryl (e.g., pyridinyl). More preferably, $R^3$ is selected from $C_{1-10}$ alkyl, $C_{2-10}$ alkenyl, or $C_{2-10}$ alkynyl, wherein said alkyl, said alkenyl or said alkynyl may be substituted with one group selected from halogen, $-CF_3$, -CN, -OH, $-O(C_{1-5}$ alkyl), -SH, $-S(C_{1-5}$ alkyl), $-NH_2$, $-NH(C_{1-5}$ alkyl), $-N(C_{1-5}$ alkyl)$(C_{1-5}$ alkyl), or phenyl. Even more preferably, $R^3$ is selected from $C_{3-8}$ alkyl, $C_{3-8}$ alkenyl, $C_{3-8}$ alkynyl, or benzyl. The alkyl, alkenyl or alkynyl is preferably linear. Yet even more preferably, $R^3$ is selected from n-hexynyl, n-heptynyl, n-octynyl, allyl, or benzyl. It is particularly preferred that $R^3$ is n-octyl.

Exemplary non-limiting compounds of formula (I) include the following compounds as well as pharmaceutically acceptable salts, solvates and prodrugs thereof:

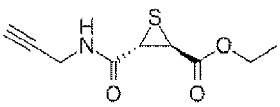

ThiEt

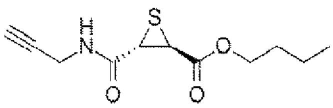

ThiBut

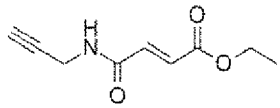

MichEt

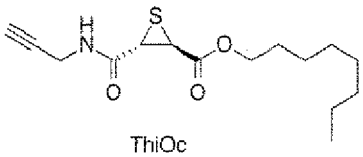

ThiOc

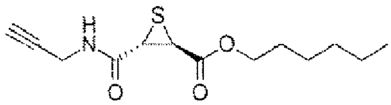

ThiHex

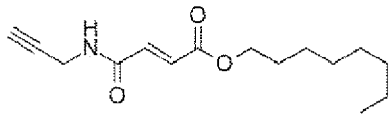

MichOc

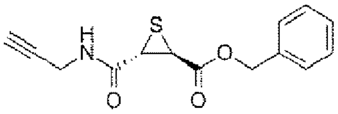

ThiBenz

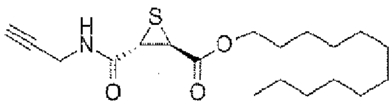

ThiDodec

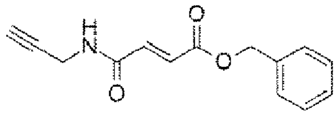

MichBenz

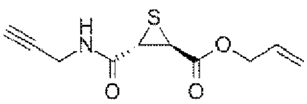

ThiAll

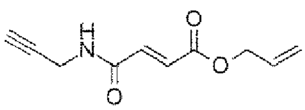

MichAll

Further exemplary non-limiting compounds of formula (I) include the compounds ThiEt, ThiBut, ThiHex, ThiOc, ThiDodec, ThiBenz, ThiAll, MichEt, Mich0c, MichBenz and MichAll as shown above, wherein the terminal carbon-to-carbon triple bond of each one of these compounds is replaced by a carbon-to-carbon single bond (i.e., the terminal group $-CH_2-C{=}CH$ is replaced by $-CH_2-CH_2-CH_3$), as well as pharmaceutically acceptable salts, solvates and prodrugs thereof.

It is preferred that the compound of formula (I) is a compound of one of the following formulae or a pharmaceutically acceptable salt, solvate or prodrug thereof:

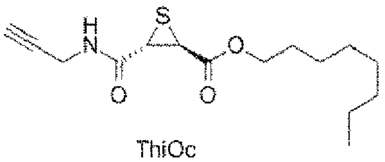

ThiOc

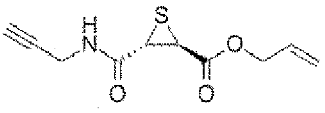

ThiAll

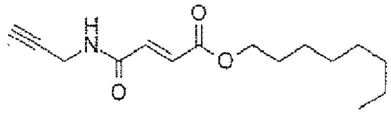

MichOc

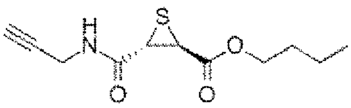

ThiBut

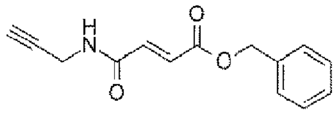

MichBenz

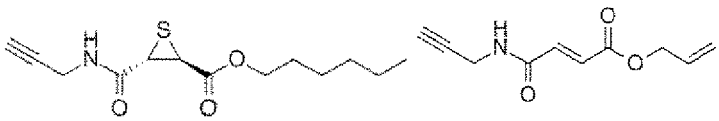

ThiHex

MichAll

Further preferred compounds of formula (I) are the above depicted compounds ThiOc, ThiAll, ThiBut, ThiHex, MichOc, MichBenz and MichAll, wherein the terminal carbon-to-carbon triple bond of each one of these compounds is replaced by a carbon-to-carbon single bond (i.e., the terminal group $-CH_2-C\equiv CH$ is replaced by $-CH_2-CH_2-CH_3$), as well as pharmaceutically acceptable salts, solvates and prodrugs thereof.

A particularly preferred compound of formula (I) is the compound "ThiOc" shown below or octyl (trans)-3-(*N*-propargylaminocarbonyl)thiirane-2-carboxylate (including, in particular, the isolated enantiomer octyl (2*R*,3*R*) 3-(*N*-propargylaminocarbonyl)thiirane-2-carboxylate, the isolated enantiomer octyl (2*S*,3*S*) 3-(*N*-propargylaminocarbonyl)thiirane-2-carboxylate, or a racemic mixture of these enantiomers, with the isolated (2*S*,3*S*) enantiomer being preferred) or a pharmaceutically acceptable salt, solvate or prodrug thereof:

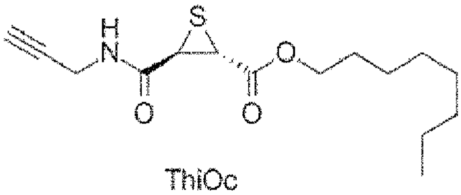

ThiOc

In one embodiment, the compound of formula (I) is a compound of the following formula (II) or a pharmaceutically acceptable salt, solvate or prodrug thereof:

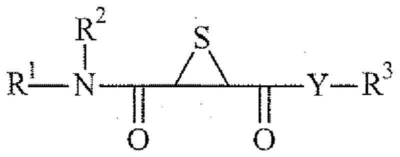

(II)

wherein the groups $R^1$ $R^2$, $R^3$ and Y have the meanings or the preferred meanings described and defined herein above for the compound of formula (I). It is furthermore preferred that the two moieties attached to the thiirane ring comprised in the compound of formula (II) - i.e., the moiety -CO-N($R^1$)$R^2$ and the moiety -CO-Y-$R^3$ - are in trans configuration.

In a further embodiment, the compound of formula (I) is a compound of the following formula (III) or a pharmaceutically acceptable salt, solvate or prodrug thereof:

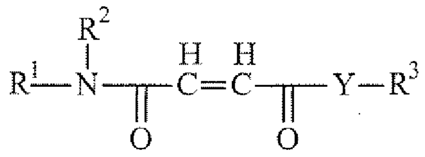

(III)

wherein the groups $R^1$, $R^2$, $R^3$ and Y have the meanings or the preferred meanings described and defined herein above for the compound of formula (I). It is furthermore preferred that the two moieties -CO-N($R^1$)$R^2$ and -CO-Y-$R^3$ which are attached to the central -CH=CH- group of the compound of formula (III) are in trans configuration.

As used herein, the term "alkyl" refers to a monovalent saturated aliphatic (i.e., non-aromatic) acyclic hydrocarbon group (i.e., a group consisting of carbon atoms and hydrogen atoms) which may be linear or branched and does not comprise any carbon-to-carbon double bond or any carbon-to-carbon triple bond. Accordingly, the term "$C_{1-4}$ alkyl" refers to methyl, ethyl, propyl (e.g., n-propyl or isopropyl), or butyl (e.g., n-butyl, isobutyl, tert-butyl, or sec-butyl).

As used herein, the term "alkenyl" refers to a monovalent unsaturated aliphatic acyclic hydrocarbon group which may be linear or branched and comprises at least one carbon-to-carbon double bond.

As used herein, the term "alkynyl" refers to a monovalent unsaturated aliphatic acyclic hydrocarbon group which may be linear or branched and comprises at least one carbon-to-carbon triple bond.

As used herein, the term "aryl" refers to a monovalent aromatic hydrocarbon group, including monocyclic as well as bridged ring and/or fused ring systems, containing at least one aromatic ring. "Aryl" may, for example, refer to phenyl, naphthyl or anthracenyl.

As used herein, the term "heteroaryl" refers to monovalent aromatic ring group, including bridged ring and/or fused ring systems, containing at least one aromatic ring and comprising one or more (such as, e.g., one, two, or three) ring heteroatoms independently selected from O, S, or N. The "heteroaryl" may, e.g., have 5 to 14 ring atoms, particularly 5 or 6 ring atoms. "heteroaryl" may, for example, refer to thiophenyl (thienyl), furanyl (furyl), pyrrolyl, imidazolyl, pyrazolyl, pyridinyl (pyridyl; including, e.g., 2-pyridyl, 3-pyridyl, and 4-pyridyl), pyrazinyl, pyrimidinyl, pyridazinyl, oxazolyl, isoxazolyl, or furazanyl.

As used herein, the term "halogen" refers to -F, -Cl, -Br, or -I.

The term "interrupted", as used, e.g., in the expression "said alkyl may be interrupted by one or more groups selected independently from...", indicates that one or more of the corresponding groups (the interrupting groups) may be present between two adjacent carbon atoms of the interrupted group (e.g., of the aforementioned alkyl group). Accordingly, an example of a $C_{1-4}$ alkyl group interrupted by -O- would be $-(CH_2)_3-O-CH_3$. If more than one interrupting group is present, it is generally preferred that each one of the interrupting groups is present between a different pair of adjacent carbon atoms of the interrupted group.

Unless indicated otherwise, in the present specification all chemical formulae showing a specific stereochemical configuration at the ring carbon atoms of a thiirane ring using the wedge-dash notation are intended to refer to (i) the corresponding cis or trans isomer as well as (ii) each one of the two possible specific stereoisomers encompassed by the respective cis or trans isomer (e.g., the (2R,3R) isomer and the (2S,3S) isomer, or the (2R,3S) isomer and the (2S, 3R) isomer). Accordingly, any formula depicting a thiirane compound according to the invention using the wedge-dash notation, including each one of the formulae of the specific compounds ThiEt, ThiBut, ThiHex, ThiOc, ThiDodec, ThiAll and ThiBenz, refers to either the cis isomer or the trans isomer (the trans isomer in the case of the aforementioned

specific thiirane compounds ThiEt, etc.) but also to each one of the two corresponding specific stereoisomers, i.e., to each one of the (2R,3R) and (2S,3S) isomers or to each one of the (2R,3S) and (2S,3R) isomers. For each one of the formulae showing a thiirane compound in the wedge-dash notation, it is preferred that the corresponding compound is present in the specific cis or trans configuration that is depicted, and it is more preferred that the compound has the specific stereochemical configuration at the ring carbon atoms of its thiirane ring moiety (i.e., (2R,3R), (2S,3S), (2R,3S) or (2S,3R)) which is shown in the respective formula.

The present invention also relates to a pharmaceutical composition comprising a compound of formula (I), (II) or (III) as defined herein or a pharmaceutically acceptable salt, solvate, or prodrug thereof, optionally in combination with a pharmaceutically acceptable excipient. Accordingly, the compounds of formula (I), (II) or (III) are useful as medicaments.

The present invention further relates to a compound of formula (I), (II) or (III) as defined herein, or a pharmaceutically acceptable salt, solvate, or prodrug thereof, or a pharmaceutical composition comprising any of the aforementioned entities optionally in combination with a pharmaceutically acceptable excipient, for use in the treatment or prevention of a bacterial infection.

Furthermore, the invention encompasses a method of treating or preventing a bacterial infection, the method comprising the administration of a compound of formula (I), (II) or (III) as defined herein, or a pharmaceutically acceptable salt, solvate, or prodrug thereof, or a pharmaceutical composition comprising any of the aforementioned entities optionally in combination with a pharmaceutically acceptable excipient, to a subject (e.g., a human) in need thereof.

The thiirane or Michael acceptor compounds according to the invention, particularly the compounds of formula (I), (II) or (III), have been found to be potent antibiotics and, thus, to be useful in the medical intervention in bacterial infections, as has been demonstrated in the examples for pathogenic bacteria such as multidrug-resistant *Staphylococcus aureus* and *Listeria monocytogenes.* In accordance therewith, the compounds of the invention have further been found to target key enzymes which are essential for bacterial viability. Accordingly, the metabolic enzymes FabF and FabH, for instance, are bound by the compound ThiOc, as also shown in the examples. Dual inhibitors of FabF and FabH are considered to effectively inhibit bacterial viability and, therefore, to be advantageous in comparison to antibacterial agents such as cerulenin which inhibits FabF but not FabH (Price, A.C. et al., J Biol Chem. 2001, 276(9): 6551-6559). The compounds of the present invention are furthermore advantageous over complex natural products such as platencin, a known dual inhibitor of FabF and FabH (Wang, J. et al., Proc Natl Acad Sci USA, 2007, 104(18): 7612-7616), as they are small molecules which can be provided more easily by chemical synthesis.

The bacterial infection to be treated or prevented using a compound or a pharmaceutical composition according to the present invention includes infections with Gram-positive or Gram-negative bacteria, particularly infections with Gram-positive bacteria.

Exemplary infections with Gram-positive bacteria include infections with bacteria of the group *Firmicutes* and infections with bacteria of the group *Actinobacteria* (*Actinomycetes*).

Gram-positive *Firmicutes* include, *inter alia,* the "*Bacilli*" (including, e.g., the genera *Bacillus*, *Listeria, Staphylococcus, Enterococcus, Lactobacillus, Lactococcus, Leuconostoc, Pediococcus*, and *Streptococcus*), the "*Clostridia*" (including, e.g., the genera *Acetobacterium*, *Clostridium*, *Eubacterium*, *Heliobacterium*, *Heliospirillum*, *Pectinatus*, and *Sporomusa*) and the "*Mollicutes*" (including, e.g., the genera *Mycoplasma*, *Spiroplasma*, *Ureaplasma*, and *Erysipelothrix*). *Actinobacteria* include, *inter alia,* the genera *Actinomyces*, *Arthrobacter, Corynebacterium, Frankia, Micrococcus, Micromonospora, Nocardia, Propionibacterium*, *Streptomyces* and *Mycobacterium.*

Non-limiting examples of bacterial species of the groups *Firmicutes* or *Actinobacteria* are *Listeria monocytogenes, Listeria ivanovii, Staphylococcus aureus, Staphylococcus epidermidis, Staphylococcus saprophyticus, Staphylococcus lugdunensis*, *Staphylococcus schleiferi, Staphylococcus caprae, Streptococcus pneumoniae, Streptococcus viridans, Streptococcus pyogenes, Streptococcus agalactiae, Enterococcus faecalis, Enterococcus faecium, Bacillus licheniformis*, *Bacillus subtilis, Bacillus anthracis, Bacillus cereus, Bacillus thuringiensis, Bacillus larvae, Mycobacterium tuberculosis, Mycobacterium leprae*, *Mycobacterium ulcerans, Mycobacterium kanasesii, Mycobacterium avium, Mycobacterium paratuberculosis, Mycobacterium scrofulaceam, Mycobacterium microti, Mycobacterium africanum, Mycobacterium canettii, Mycobacterium intracellulare, Mycobacterium simiae, Mycobacterium szulgai, Mycobacterium xenopi, Mycobacterium fortuitum, Mycobacterium chelonei*, *Mycobacterium marinum*, *Nocardia asteroides*, and *Rhodococcus equi.*

The present invention relates to the treatment or prevention of a bacterial infection with any of the above described bacteria using a compound of formula (I), (II) or (III). Accordingly, the compounds of the present invention are useful in the treatment or prevention of an infection with Gram-positive bacteria, particularly *Firmicutes* or *Actinobacteria*, including, e.g., *Staphylococcus* (particularly *S. aureus*) or *Listeria* (particularly *L. monocytogenes)*.

The compounds of the present invention can also be used for treating or preventing an infection with multidrug-resistant bacteria, particularly multidrug-resistant Gram-positive bacteria, such as, e.g., multidrug-resistant (or methicillin-resistant) *Staphylococcus aureus* (MRSA), vancomycin intermediate *Staphylococcus aureus* (VISA), or vancomycin resistant *Staphylococcus aureus* (VRSA).

Gram-negative bacteria include, *inter alia,* the genera *Escherichia*, *Salmonella*, *Pseudomonas, Moraxella, Helicobacter, Stenotrophomonas*, *Bdellovibrio, Legionella, Vibrio, Neisseria, Hemophilus, Chlamydia, Klebsiella, Legionella, Proteus, Enterobacter,* and *Serratia.* Non-limiting examples of Gram-negative bacterial species are *Neisseria gonorrhoeae, Neisseria meningitidis, Moraxella catarrhalis, Haemophilus influenzae, Klebsiella pneumoniae, Legionella pneumophila, Pseudomonas aeruginosa, Pseudomonas putida, Escherichia coli* (including, e.g., enterohemorrhagic *E. coli* (EHEC), enterotoxigenic *E. coli* (ETEC), enteropathogenic *E. coli* (EPEC), enteroinvasive *E. coli* (EIEC), enteroaggregative *E. coli* (EAEC), and diffusely adherent *E. coli* (DAEC)), *Proteus mirabilis, Enterobacter cloaceae, Serratia marcescens, Helicobacter pylori, Salmonella enteritidis,* and *Salmonella typhi.* It is envisaged that the compounds of the present invention, including the compounds of formula (I), (II) or (III), are to be used for treating or preventing infections with Gram-negative bacteria, including, e.g., infections with any of the above described Gram-negative bacteria, and particularly infections with bacteria of the group *Proteobacteria.*

Thus, the present invention particularly relates to a compound of formula (I), (II) or (III) or a pharmaceutically acceptable salt, solvate or prodrug thereof, or a pharmaceutical composition comprising any of the aforementioned entities and optionally a pharmaceutically acceptable excipient, for use in the treatment of prevention of an infection with *Listeria monocytogenes, Listeria ivanovii, Staphylococcus aureus, Staphylococcus epidermidis, Staphylococcus saprophyticus, Staphylococcus lugdunensis, Staphylococcus schleiferi, Staphylococcus caprae, Streptococcus pneumoniae, Streptococcus viridans, Streptococcus pyogenes, Streptococcus agalactiae, Enterococcus faecalis, Enterococcus faecium, Bacillus licheniformis, Bacillus subtilis, Bacillus anthracis, Bacillus cereus, Bacillus thuringiensis, Bacillus larvae, Mycobacterium tuberculosis, Mycobacterium leprae, Mycobacterium ulcerans, Mycobacterium kanasasii, Mycobacterium avium, Mycobacterium paratuberculosis, Mycobacterium scrofulaceam, Mycobacterium microti, Mycobacterium africanum, Mycobacterium canettii, Mycobacterium intracellulare, Mycobacterium simiae, Mycobacterium szulgai, Mycobacterium xenopi, Mycobacterium fortuitum, Mycobacterium chelonei, Mycobacterium marinum, Nocardia asteroides, Rhodococcus equi, Neisseria gonorrhoeae, Neisseria meningitidis, Moraxella catarrhalis, Haemophilus influenzae, Klebsiella pneumoniae, Legionella pneumophila, Pseudomonas aeruginosa, Pseudomonas putida, Escherichia coli, Proteus mirabilis, Enterobacter cloaceae, Serratia marcescens, Helicobacter pylori, Salmonella enteritidis,* or *Salmonella typhi,* particularly an infection with *Staphylococcus aureus* or *Listeria monocytogenes.*

The treatment or prevention of a bacterial infection, as referred to herein, also includes the treatment or prevention of a disease induced by and/or related to a bacterial infection. Accordingly, the present invention also relates to the treatment or prevention of a bacterial infection or an infectious disease selected from listeriosis, cranial nerve palsy, encephalitis, meningitis, meningoencephalitis, abscesses (including, e.g., brain abscesses, lung abscesses and kidney abscesses), endocarditis, pneumonia, cholera, syphilis, diphtheria, anthrax, leprosy, tuberculosis, bubonic plague, sepsis, septic arthritis, osteitis, inflammation of wounds, furuncles, carbuncles, toxic shock syndrome, Staphylococcal scalded skin syndrome (Ritter's disease), or mastitis.

The compounds of the present invention, including the compounds of formula (I), (II) or (III), can be prepared by methods known in the field of synthetic chemistry. For example, these compounds can be prepared in accordance with or in analogy to the synthetic routes described in the examples.

One exemplary synthetic route is described in the following. All chemicals were of reagent grade or better and used without further purification. Chemicals and solvents were purchased from Sigma Aldrich. (+)-Diethyl L-tartrate was converted to diethyl (2S,3*R*) 2-bromo-3-hydroxy-butanedioate by the method of *Mori.* (Mori, K., Iwasawa, H. Tetrahedron 1980, 87-90). The bromohydrin was converted to diethyl (*2*R,3*R*) oxirane-2,3-dicarboxylate by treatment with NaOEt (Meara, J. P.; Rich, D. H. J Med Chem 1996, 39 ,6, 3357-3366). The diester was converted into the monoester by addition of KOH in abs. EtOH. The monoester was dissolved in dry dichloromethane (DCM) and mixed with *N*,*N'*-diisopropylcarbodiimide (DIC) and propargylamine which led to the corresponding amide (OxyEt). Basic hydrolysis of the second ethylester in EtOH gave the free acid which could be converted to a variety of esters by using mixed anhydrides as activation reagents. The thiiranes could be obtained by transformation of the corresponding oxiranes by reaction with triphenylphosphine sulfide based on the procedure published by *Korn* (Korn, A. C., Moroder L. J Chem Res (S) 1995, 102-103). Further compounds according the invention can be prepared in analogy to this synthetic route.

The scope of the invention embraces all pharmaceutically acceptable salt forms of the compounds of the present invention, in particular the compounds of formula (I), (II) or (III), which may be formed, e.g., by protonation of an atom carrying an electron lone pair which is susceptible to protonation, such as an amino group, with an inorganic or organic acid. Exemplary acid addition salts comprise, for example, mineral acid salts such as hydrochloride, hydrobromide, hydroiodide, sulfate salts, nitrate salts, phosphate salts (such as, e.g., phosphate, hydrogenphasphate, or dihydrogenphosphate salts), carbonate salts, hydrogencarbonate salts or perchlorate salts; organic acid salts such as acetate, propionate, butyrate, pentanoate, hexanoate, heptanoate, octanoate, cyclopentanepropionate, undecanoate, lactate, maleate, oxalate, fumarate, tartrate, malate, citrate, nicotinate, benzoate, salicylate or ascorbate salts; sulfonate salts such as methanesulfonate, ethanesulfonate, 2-hydroxyethanesulfonate, benzenesulfonate, p-toluenesulfonate (tosylate), 2-naphthalenesulfonate, 3-phenylsulfonate, or camphorsulfonate salts; and acidic amino acid salts such as aspartate or glutamate salts.

Moreover, the scope of the invention embraces the compounds of formula (I), (II) or (III) in any solvated form, including, e.g., solvates with water, for example hydrates, or with organic solvents such as, e.g., methanol, ethanol or acetonitrile, i.e., as a methanolate, ethanolate or acetonitrilate, respectively, or in the form of any polymorph.

Furthermore, the formulae in the present application are intended to cover all possible stereoisomers, including enantiomers and diastereomers, of the indicated compounds.

Thus, all stereoisomers of the compounds of formula (I), (II) or (III) are contemplated as part of the present invention, either in admixture or in pure or substantially pure form. The scope of the compounds according to the invention embraces all of the possible stereoisomers and their mixtures. It particularly embraces the racemic forms and the isolated optical isomers. The racemic forms can be resolved by physical methods, such as, e.g., fractional crystallization, separation or crystallization of diastereomeric derivatives or separation by chiral column chromatography. The individual optical isomers can be obtained from the racemates using conventional methods, such as, e.g., salt formation with an optically active acid followed by crystallization.

Pharmaceutically acceptable prodrugs of the compounds of formula (I), (II) or (III) are derivatives which have chemically or metabolically cleavable groups and become, by solvolysis or under physiological conditions, the compounds of the present invention which are pharmaceutically active *in vivo.* Prodrugs of the compounds of formula (I), (II) or (III) may be formed in a conventional manner with a functional group of the compounds such as with an amino or a hydroxyl group. The prodrug derivative form often offers advantages of solubility, tissue compatibility or delayed release in a mammalian organism (see, Bundgaard, H., Design of Prodrugs, pp. 7-9, 21-24, Elsevier, Amsterdam 1985). When a compound employed in the present invention has a hydroxyl group, an acyloxy derivative prepared by reacting the hydroxyl group with a suitable acylhalide or a suitable acid anhydride is exemplified as a prodrug. An especially preferred acyloxy derivative as a prodrug is $-OC(=O)-CH_3$, $-OC(=O)-C_2H_5$, -OC(=O)-(tert-Bu), $-OC(=O)-C_{15}H_{31}$, -OC(=O)-(m-COONa-Ph), $-OC(=O)-CH_2CH_2COONa$, $-O(C=O)-CH(NH_2)CH_3$ or $-OC(=O)-CH_2-N(CH_3)_2$. When a compound employed in the present invention has an amino group, an amide derivative prepared by reacting the amino group with a suitable acid halide or a suitable mixed anhydride is exemplified as a prodrug. An especially preferred amide derivative as a prodrug is $-NHC(=O)-(CH_2)_2OCH_3$ or $-NHC(=O)-CH(NH_2)CH_3$.

The compounds described herein may be administered as compounds *per se* or may be formulated as medicaments. The medicaments/pharmaceutical compositions may optionally comprise one or more pharmaceutically acceptable excipients, such as carriers, diluents, fillers, disintegrants, lubricating agents, binders, colorants, pigments, stabilizers, preservatives, antioxidants, or solubility enhancers.

In particular, the pharmaceutical compositions may comprise one or more solubility enhancers, such as, e.g., poly(ethylene glycol), including poly(ethylene glycol) having a molecular weight in the range of about 200 to about 5,000 Da, ethylene glycol, propylene glycol, non-ionic surfactants, tyloxapol, polysorbate 80, macrogol-15-hydroxystearate, phospholipids, lecithin, dimyristoyl phosphatidylcholine, dipalmitoyl phosphatidylcholine, distearoyl phosphatidylcholine, cyclodextrins, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, glucosyl-α-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, carboxyalkyl thioethers, hydroxypropyl methylcellulose, hydroxypropylcellulose, polyvinylpyrrolidone, vinyl acetate copolymers, vinyl pyrrolidone, sodium lauryl sulfate, dioctyl sodium sulfosuccinate, or any combination thereof.

The pharmaceutical compositions can be formulated by techniques known to the person skilled in the art, such as the techniques published in Remington's Pharmaceutical Sciences, 20th Edition. The pharmaceutical compositions can be formulated as dosage forms for oral, parenteral, such as intramuscular, intravenous, intraperitoneal, subcutaneous, intradermal, intraarterial, rectal, nasal, topical, aerosol or vaginal administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insufflation, for example by a metered inhaler. Dosage forms for topical administration include creams, gels, ointments, salves, patches and transdermal delivery systems.

The compounds of formula (I), (II) or (III) or the above described pharmaceutical compositions comprising a compound of formula (I), (II) or (III) may be administered to a subject by any convenient route of administration, whether systemically/peripherally or at the site of desired action, including but not limited to one or more of: oral (e.g., as a tablet, capsule, or as an ingestible solution), topical (e.g., transdermal, intranasal, ocular, buccal, and sublingual), parenteral (e.g., using injection techniques or infusion techniques, and including, for example, by injection, e.g., subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrasternal by, e.g., implant of a

depot, for example, subcutaneously or intramuscularly), pulmonary (e.g., by inhalation or insufflation therapy using, e.g., an aerosol, e.g., through mouth or nose), gastrointestinal, intrauterine, intraocular, subcutaneous, ophthalmic (including intravitreal or intracameral), rectal, and vaginal.

If the compounds or pharmaceutical compositions are administered parenterally, then examples of such administration include one or more of: intravenously, intraarterially, intraperitoneally, intrathecally, intraventricularly, intraurethrally, intrasternally, intracranially, intramuscularly or subcutaneously administering the compounds pharmaceutical compositions, and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

The compounds or pharmaceutical compositions can also be administered orally in the form of tablets, capsules, ovules, elixirs, solutions or suspensions, which may contain flavoring or coloring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

Alternatively, the compounds or pharmaceutical compositions can be administered in the form of a suppository or pessary, or it may be applied topically in the form of a gel, hydrogel, lotion, solution, cream, ointment or dusting powder. The compounds of the present invention may also be dermally or transdermally administered, for example, by the use of a skin patch.

The compounds or pharmaceutical compositions may also be administered by the pulmonary route, rectal routes, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

It is furthermore envisaged to prepare dry powder formulations of the compounds of the present invention for pulmonary administration, particularly inhalation. The dry powder formulations of the compounds of the present invention may be delivered using any suitable dry powder inhaler (DPI), i.e., an inhaler device that utilizes the patient's inhaled breath as a vehicle to transport the dry powder drug to the lungs.

For topical application to the skin, the compounds or pharmaceutical compositions can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

Typically, a physician will determine the actual dosage which will be most suitable for an individual subject. The specific dose level and frequency of dosage for any particular individual subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual subject undergoing therapy.

A proposed, yet non-limiting dose of the compounds of the invention, including the compounds of formula (I), (II) or (III), for administration to a human (of approximately 70 kg body weight) may be about 0.1 $\mu$g to about 10 g, preferably about 0.1 mg to about 1 g, and more preferably about 200 mg to about 500 mg, of the active ingredient per unit dose. The unit dose may be administered, for example, 1 to 3 times per day. The unit dose may also be administered 1 to 7 times per week, e.g., with not more than one administration per day. The dose will depend on the route of administration. It will be appreciated that it may be necessary to make routine variations to the dosage depending on the age and weight of the patient/subject as well as the severity of the condition to be treated. The precise dose and route of administration will ultimately be at the discretion of the attendant physician or veterinarian.

The compounds according to the present invention can also be used in combination with other therapeutic agents. When a compound of the invention is used in combination with a second therapeutic agent active against the same disease, the dose of each compound may differ from that when the compound is used alone. The combination of

a compound of the invention with one or more other drugs may comprise the simultaneous/concomitant administration of the other drug or drugs with the compound of the invention or the sequential/separate administration.

Preferably, the second therapeutic agent to be administered in combination with a compound of the present invention is an antibiotic or an antiseptic. Accordingly, it is envisaged that one or more compounds according to the present invention, particularly a compound of formula (I), (II) or (III), are to be used in combination with one or more antibiotics and/or one or more antiseptics.

The one or more antibiotics to be used in combination with a compound of the invention include, without being limited thereto: tetracycline-derived antibiotics such as, e.g., tetracycline, doxycycline, chlortetracycline, clomocycline, demeclocycline, lymecycline, meclocycline, metacycline, minocycline, oxytetracycline, penimepicycline, rolitetracycline, or tigecycline; amphenicol-derived antiobiotics such as, e.g., chloramphenicol, azidamfenicol, thiamphenicol, or florfenicol; macrolide-derived antiobiotics such as, e.g., erythromycin, azithromycin, spiramycin, midecamycin, oleandomycin, roxithromycin, josamycin, troleandomycin, clarithromycin, miocamycin, rokitamycin, dirithromycin, flurithromycin, telithromycin, cethromycin, tulathromycin, carbomycin A, kitasamycin, midecamicine, midecamicine acetate, or tylosin (tylocine); ketolide-derived antibiotics such as, e.g., telithromycin, or cethromycin; lincosamide-derived antiobiotics such as, e.g., clindamycin, or lincomycin; streptogramin-derived antiobiotics such as, e.g., pristinamycin, or quinupristin/dalfopristin; oxazolidinone-derived antiobiotics such as, e.g., linezolid, or cycloserine; aminoglycoside-derived antiobiotics such as, e.g., streptomycin, neomycin, framycetin, paromomycin, ribostamycin, kanamycin, amikacin, arbekacin, bekanamycin, dibekacin, tobramycin, spectinomycin, hygromycin B, paromomycin, gentamicin, netilmicin, sisomicin, isepamicin, verdamicin, astromicin, rhodostreptomycin, or apramycin; steroid-derived antiobiotics such as, e.g., fusidic acid, or sodium fusidate; glycopeptide-derived antiobiotics such as, e.g., vancomycin, oritavancin, telavancin, teicoplanin, dalbavancin, ramoplanin, bleomycin, or decaplanin; beta-lactam-derived antiobiotics such as, e.g., amoxicillin, ampicillin, pivampicillin, hetacillin, bacampicillin, metampicillin, talampicillin, epicillin, carbenicillin, carindacillin, ticarcillin, temocillin, azlocillin, piperacillin, mezlacillin, mecillinam, pivmecillinam, sulbenicillin, benzylpenicillin, azidocillin, penamecillin, clometocillin, benzathine benzylpenicillin, procaine benzylpenicillin, phenoxymethylpenicillin, propicillin, benzathine, phenoxymethylpenicillin, pheneticillin, axacillin, ciaxacillin, dicloxacillin, flucloxacillin, meticillin, nafcillin, faropenem, biapenem, doripenem, ertapenem, imipenem, meropenem, panipenem, cefacetrile, cefadroxil, cefalexin, cefaloglycin, cefalonium, cefaloridine, cefalotin, cefapirin, cefatrizine, cefazedone, cefazaflur, cefazolin, cefradine, cefroxadine, ceftezole, cefaclor, cefamandole, cefminox, cefonicid, ceforanide, cefotiam, cefprozil, cefbuperazone, cefuroxime, cefuzonam, cefoxitin, cefotetan, cefmetazole, loracarbef, cefcapene, cefdaloxime, cefdinir, cefditoren, cefetamet, cefixime, cefmenoxime, cefodizime, cefoperazone, cefotaxime, cefpimizole, cefpiramide, cefpodoxime, cefsulodin, ceftazidime, cefteram, ceftibuten, ceftiolene, ceftizoxime, ceftriaxone, flomoxef, latamoxef, cefepime, cefozopran, cefpirome, cefquinome, ceftobiprole, aztreonam, tigemonam, sulbactam, tazobactam, clavulanic acid, ampicillin/sulbactam, sultamicillin, piperacillin/tazobactam, co-amoxiclav, amoxicillin/clavulanic acid, or imipenem/cilastatin; sulfonamide-derived antiobiotics such as, e.g., acetazolamide, benzolamide, bumetanide, celecoxib, chlorthalidone, clopamide, dichlorphenamide, dorzolamide, ethoxzolamide, furosemide, hydrochlorothiazide, indapamide, mafenide, mefruside, metolazone, probenecid, sulfacetamide, sulfadiazine, sulfadimethoxine, sulfadoxine, sulfanilamides, sulfamethoxazole, sulfamethoxypyridazine, sulfasalazine, sultiame, sumatriptan, xipamide, zonisamide, sulfaisodimidine, sulfamethizole, sulfadimidine, sulfapyridine, sulfafurazole, sulfathiazole, sulfathiourea, sulfamoxole, sulfadimethoxine, sulfalene, sulfametomidine, sulfametoxydiazine, sulfaperin, sulfamerazine, sulfaphenazole, or sulfamazone; quinolone-derived antiobiotics such as, e.g., cinoxacin, flumequine, nalidixic acid, oxolinic acid, pipemidic acid, piromidic acid, rosoxacin, ciprofloxacin, enoxacin, fleroxacin, lomefloxacin, nadifloxacin, ofloxacin, norfloxacin, pefloxacin, rufloxacin, balofloxacin, grepafloxacin, levofloxacin, pazufloxacin, sparfloxacin, temafloxacin, tosufloxacin, besifloxacin, clinafloxacin, garenoxacin, gemifloxacin, moxifloxacin, gatifloxacin, sitafloxacin, trovafloxacin, alatrofloxacin, prulifloxacin, danofloxacin, difloxacin, enrofloxacin, ibafloxacin, marbofloxacin, orbifloxacin, pradofloxacin, sarafloxacin, ecinofloxacin, or delafloxacin; imidazole-derived antiobiotics such as, e.g., metronidazole; nitrofuran-derived antiobiotics such as, e.g., nitrofurantoin, or nifurtoinol; aminocoumarin-derived antiobiotics such as, e.g., novobiocin, clorobiocin, or coumermycin A1; ansamycin-derived antiobiotics, including rifamycin-derived antiobiotics such as, e.g., rifampicin (rifampin), rifabutin, rifapentine, or rifaximin; and also further antiobiotics such as, e.g., fosfomycin, bacitracin, colistin, polymyxin B, daptomycin, xibornol, clofoctol, methenamine, mandelic acid, nitroxoline, mupirocin, trimethoprim, brodimoprim, iclaprim, tetroxoprim, or sulfametrole.

The one or more antiseptics to be used in combination with a compound of the invention include, without being limited thereto: acridine-derived antiseptics such as, e.g., ethacridine lactate, aminoacridine, or euflavine; amidine-derived or biguanide-derived antiseptics such as, e.g., dibrompropamidine, chlorhexidine, propamidine, hexamidine, or polihexanide; phenol-derived antiseptics such as, e.g., phenol, hexachlorophene, policresulen, triclosan, chloroxylenol, or biphenylol; nitrofuran-derived antiseptics such as, e.g., nitrofurazone; iodine-based antiseptics such as, e.g., iodine/octylphenoxypolyglycolether, povidone-iodine, or diiodohydroxypropane; quinoline-derived antiseptics such as, e.g., dequalinium, chlorquinaldol, oxyquinoline, or clioquinol; quaternary ammonium-derived antiseptics such as, e.g., benzalkonium, cetrimonium, cetylpyridinium, cetrimide, benzoxonium chloride, or didecyldimethylammonium chloride; mercurial antiseptics such as, e.g., mercuric amidochloride, phenylmercuric borate, mercuric chloride, mercurochrome,

thiomersal, or mercuric iodide; silver-based antiseptics such as, e.g., silver nitrate; alcoholic antiseptics such as, e.g., propanol (including isopropanol), or ethanol; and also further antiseptics such as, e.g., potassium permanganate, sodium hypochlorite, hydrogen peroxide, eosin, tosylchloramide sodium, dichlorobenzyl alcohol, ambazone, benzethonium, myristyl-benzalkonium, hexylresorcinol, or acriflavinium chloride.

The combinations referred to above may conveniently be presented for use in the form of a pharmaceutical formulation. The individual components of such combinations may be administered either sequentially or simultaneously/concomitantly in separate or combined pharmaceutical formulations by any convenient route. When administration is sequential, either the present compound or the second therapeutic agent may be administered first. When administration is simultaneous, the combination may be administered either in the same or different pharmaceutical composition. When combined in the same formulation it will be appreciated that the two compounds must be stable and compatible with each other and the other components of the formulation. When formulated separately they may be provided in any convenient formulation, conveniently in such manners as are known for such compounds in the art.

Accordingly, in one embodiment, the present invention relates to a compounds of formula (I), (II) or (III) or a pharmaceutically acceptable salt, solvate, or prodrug thereof, or a pharmaceutical composition comprising any of the aforementioned entities and a pharmaceutically acceptable excipient, for use in the treatment or prevention of a bacterial infection, wherein the compound or the pharmaceutical composition is to be administered in combination with one or more antibiotics and/or one or more antiseptics.

The subject or patient, such as the subject in need of treatment or prophylaxis, may be an animal, a vertebrate animal, a mammal, a rodent (e.g., a guinea pig, a hamster, a rat, a mouse), a murine (e.g., a mouse), a canine (e.g., a dog), a feline (e.g., a cat), an equine (e.g., a horse), a primate, a simian (e.g., a monkey or ape), a monkey (e.g., a marmoset, a baboon), an ape (e.g., a gorilla, chimpanzee, orangutan, gibbon), or a human. The meaning of the terms "animal", "mammal", etc. is well known in the art and can, for example, be deduced from Wehner und Gehring (1995; Thieme Verlag). In the context of this invention, it is particularly envisaged that animals are to be treated which are economically, agronomically or scientifically important. Scientifically important organisms include, but are not limited to, mice, rats, and rabbits. Lower organisms such as, e.g., fruit flies like *Drosophila melagonaster* and nematodes like *Caenorhabditis elegans* may also be used in scientific approaches. Non-limiting examples of agronomically important animals are sheep, cattle and pigs, while, for example, cats and dogs may be considered as economically important animals. The treatment of insects, in particular economically important insects, such as the honey bee, is also envisaged. Preferably, the subject/patient is a mammal. More preferably, the subject/patient is a human.

The term "treatment of a disorder or disease" as used herein, e.g., in the case of the treatment of a bacterial infection, is well known in the art. "Treatment of a disorder or disease" implies that a disorder or disease has been diagnosed in a patient/subject. A patient/subject suspected of suffering from a disorder or disease typically shows specific clinical and/or pathological symptoms which a skilled person can easily attribute to a specific pathological condition (i.e., make a diagnosis of a disorder or disease).

"Treatment of a disorder or disease" may, for example, lead to a halt in the progression of the disorder or disease (e.g., no deterioration of symptoms) or a delay in the progression of the disorder or disease (in case the halt in progression is of a transient nature only). "Treatment of a disorder or disease" may also lead to a partial response (e.g., amelioration of symptoms) or complete response (e.g., disappearance of symptoms) of the subject/patient suffering from the disorder or disease. "Amelioration" of a disorder or disease may, for example, lead to a halt in the progression of the disorder or disease or a delay in the progression of the disorder or disease. Such a partial or complete response may be followed by a relapse. It is to be understood that a subject/patient may experience a broad range of responses to a treatment (e.g., the exemplary responses as described herein above).

Treatment of a disorder or disease may, inter alia, comprise curative treatment (preferably leading to a complete response and eventually to healing of the disorder or disease) and palliative treatment (including symptomatic relief).

Also the term "prevention of a disorder or disease" as used herein, e.g., in the case of the prevention of a bacterial infection, is well known in the art. For example, a patient/subject suspected of being prone to suffer from a disorder or disease as defined herein may, in particular, benefit from a prevention of the disorder or disease. The subject/patient may have a susceptibility or predisposition for a disorder or disease, including but not limited to hereditary predisposition. Such a predisposition can be determined by standard assays, using, for example, genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of compounds of the present invention before any clinical and/or pathological symptoms are diagnosed or determined or can be diagnosed or determined by the attending physician.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The invention particularly encompasses the following items:

1. A compound of formula (I)

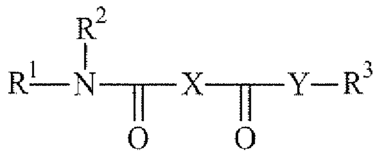

(I)

wherein:
X is

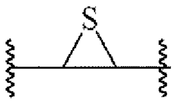

or

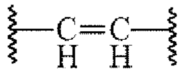

;

Y is -O- or -S-;
$R^1$ is selected from $C_{2-8}$ alkynyl, $C_{1-8}$ alkyl, or $C_{2-8}$ alkenyl, wherein said alkynyl, said alkyl or said alkenyl is optionally interrupted by one or more groups independently selected from -O-, -S-, -N(H)-, -N($C_{1-5}$ alkyl)-, -C(=O)-, -C(=O)-N(H)-, -C(=O)-N($C_{1-5}$ alkyl)-, -N(H)-C(=O)-, or -N($C_{1-5}$ alkyl)-C(=O)-, and further wherein said alkynyl, said alkyl or said alkenyl is optionally substituted with one or more groups independently selected from halogen, $-CF_3$, -CN, -OH, - O($C_{1-5}$ alkyl), -SH, -S($C_{1-5}$ alkyl), $-NH_2$, -NH($C_{1-5}$ alkyl), -N($C_{1-5}$ alkyl)($C_{1-5}$ alkyl), -CO-($C_{1-5}$ alkyl), $-CO-NH_2$, -CO-NH-($C_{1-5}$ alkyl), -CO-N($C_{1-6}$ alkyl)($C_{1-6}$ alkyl), -NH-CO-($C_{1-5}$ alkyl), -N($C_{1-5}$ alkyl)-CO-($C_{1-5}$ alkyl), aryl, or heteroaryl;
$R^2$ is H or $C_{1-5}$ alkyl; and
$R^3$ is selected from $C_{1-14}$ alkyl, $C_{2-14}$ alkenyl, or $C_{2-14}$ alkynyl, wherein said alkyl, said alkenyl or said alkynyl is optionally interrupted by one or more groups independently selected from -O-, -S-, -N(H)-, -N($C_{1-5}$ alky)-, -C(=O)-, -C(=O)-N(H)-, -C(=O)-N($C_{1-5}$ alkyl)-, -N(H)-C(=O)-, or -N($C_{1-5}$ alkyl)-C(=O)-, and further wherein said alkyl, said alkenyl or said alkynyl is optionally substituted with one or more groups independently selected from halogen, $-CF_3$, -CN, -OH, -O($C_{1-5}$ alkyl), -SH, -S($C_{1-5}$ alkyl), $-NH_2$, -NH($C_{1-5}$ alkyl), -N($C_{1-5}$ alkyl)($C_{1-5}$ alkyl), -CO-($C_{1-5}$ alkyl), $-CO-NH_2$, -CO-NH-($C_{1-5}$ alkyl), -CO-N($C_{1-5}$ alkyl)($C_{1-5}$ alkyl), -NH-CO-($C_{1-5}$ alkyl), or -N($C_{1-5}$ alkyl)-CO-($C_{1-5}$ alkyl), aryl, or heteroaryl;
or a pharmaceutically acceptable salt, solvate or prodrug thereof.

2. The compound of item 1, wherein X is

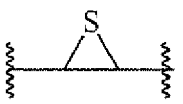

.

3. The compound of item 1, wherein X is

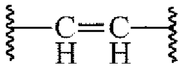

.

4. The compound of any of items 1 to 3, wherein Y is -O-.

5. The compound of any of items 1 to 4, wherein $R^1$ is selected from $C_{1-8}$ alkyl, $C_{2-8}$ alkenyl, or $C_{2-8}$ alkynyl, wherein said alkyl, said alkenyl or said alkynyl is optionally interrupted by one or more -O- groups, and further wherein said alkyl, said alkenyl or said alkynyl is optionally substituted with one or more groups independently selected from

halogen, $-CF_3$, -CN, -OH, -O($C_{1-5}$ alkyl), -SH, -S($C_{1-5}$ alkyl), $-NH_2$, -NH($C_{1-5}$ alkyl), or -N($C_{1-5}$ alkyl)($C_{1-5}$ alkyl).

6. The compound of any of items 1 to 5, wherein $R^2$ is H.

7. The compound of any of items 1 to 6, wherein $R^3$ is selected from $C_{1-10}$ alkyl, $C_{2-10}$ alkenyl, or $C_{2-10}$ alkynyl, wherein said alkyl, said alkenyl or said alkynyl is optionally substituted with one group selected from halogen, $-CF_3$, -CN, -OH, -O($C_{1-5}$ alkyl), -SH, -S($C_{1-5}$ alkyl), $-NH_2$, -NH($C_{1-5}$ alkyl), -N($C_{1-5}$ alkyl)($C_{1-5}$ alkyl), or phenyl.

8. The compound of any of items 1 to 7, wherein $R^3$ is selected from n-hexynyl, n-heptynyl, n-octynyl, allyl, or benzyl.

9. The compound of item 1, wherein the compound is a compound selected from:

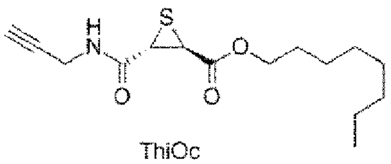

ThiOc

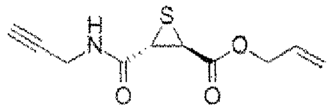

ThiAll

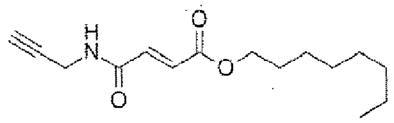

MichOc

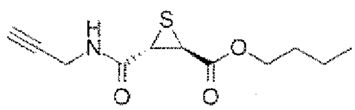

ThiBut

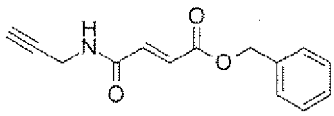

MichBenz

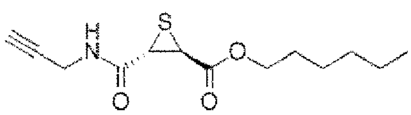

ThiHex

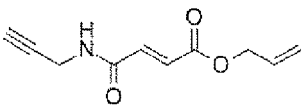

MichAll

or a pharmaceutically acceptable salt, solvate or prodrug thereof.

10. A pharmaceutical composition comprising the compound of any of items 1 to 9 and optionally a pharmaceutically acceptable excipient.

11. The compound of any of items 1 to 9 or the pharmaceutical composition of item 10 for use in treating or preventing a bacterial infection.

12. A method of treating or preventing a bacterial infection, the method comprising administering the compound of any of items 1 to 9 or the pharmaceutical composition of item 10 to a subject in need thereof.

13. The compound of item 11 or the pharmaceutical composition of item 11 or the method of item 12, wherein the bacterial infection is caused by Gram-positive bacteria.

14. The compound of item 13 or the pharmaceutical composition of item 13 or the method of item 13, wherein the Gram-positive bacteria are *Firmicutes* or *Actinobacteria.*

15. The compound of any of items 11, 13 or 14 or the pharmaceutical composition of any of items 11, 13 or 14 or

the method of any of items 12 to 14, wherein the bacterial infection is caused by *Listeria monocytogenes, Listeria ivanovii, Staphylococcus aureus, Staphylococcus epidermidis, Staphylococcus saprophyticus, Staphylococcus lugdunensis, Staphylococcus schleiferi, Staphylococcus caprae, Streptococcus pneumoniae, Streptococcus viridans, Streptococcus pyogenes, Streptococcus agalactiae, Enterococcus faecalis, Enterococcus faecium, Bacillus licheniformis*, *Bacillus subtilis, Bacillus anthracis, Bacillus cereus, Bacillus thuringiensis, Bacillus larvae, Mycobacterium tuberculosis, Mycobacterium leprae, Mycobacterium ulcerans, Mycobacterium kanasasii, Mycobacterium avium, Mycobacterium paratuberculosis, Mycobacterium scrofulaceam*, *Mycobacterium microti, Mycobacterium africanum, Mycobacterium canettii, Mycobacterium intracellulare, Mycobacterium simiae*, *Mycobacterium szulgai, Mycobacterium xenopi*, *Mycobacterium fortuitum, Mycobacterium chelonei, Mycobacterium marinum, Nocardia asteroides,* or *Rhodococcus equi.*

16. The compound of any of items 11 or 13 to 15 or the pharmaceutical composition of any of items 11 or 13 to 15 or the method of any of items 12 to 15, wherein the bacterial infection is caused by multidrug-resistant *Staphylococcus aureus.*

17. The compound of item 11 or the pharmaceutical composition of item 11 or the method of item 12, wherein the bacterial infection is caused by Gram-negative bacteria.

18. The compound of item 17 or the pharmaceutical composition of item 17 or the method of item 17, wherein the bacterial infection is caused by *Neisseria gonorrhoeae*, *Neisseria meningitidis, Moraxella catarrhalis, Haemophilus influenzae*, *Klebsiella pneumoniae, Legionella pneumophila, Pseudomonas aeruginosa, Pseudomonas putida, Escherichia coli, Proteus mirabilis, Enterobacter cloaceae, Serratia marcescens*, *Helicobacter pylori*, *Salmonella enteritidis*, or *Salmonella typhi.*

19. The compound of item 11 or the pharmaceutical composition of item 11 or the method of item 12, wherein the bacterial infection is selected from listeriosis, cranial nerve palsy, encephalitis, meningitis, meningoencephalitis, abscesses, endocarditis, pneumonia, cholera, syphilis, diphtheria, anthrax, leprosy, tuberculosis, bubonic plague, sepsis, septic arthritis, osteitis, inflammation of wounds, furuncles, carbuncles, toxic shock syndrome, Staphylococcal scalded skin syndrome, or mastitis.

20. The compound of any of items 11 or 13 to 19 or the pharmaceutical composition of any of items 11 or 13 to 19 or the method of any of items 12 to 19, wherein the compound or the pharmaceutical composition is to be administered in combination with one or more antibiotics and/or one or more antiseptics.

The invention is also illustrated by the following illustrative figures. The appended figures show:

**Figure 1:** Principles of activity based protein profiling using a rhodamine azide tag under click chemistry conditions ("TCEP" refers to tris(carboxyethyl)phosphine).

**Figure 2:** *S. aureus* proteome labeling with the compounds ThiOc and ThiBenz.

**Figure 3:** *L. welshimeri* proteome labeling with the compounds ThiOc and OxyOc.

The invention will now be described by reference to the following examples which are merely illustrative and are not to be construed as a limitation of the scope of the present invention.

**Examples**

<u>Design and-sythesis of three-membered heterocyclic probes and corresponding Michael acceptor probes</u>

A comprehensive library of functionalized oxirane, thiirane and aziridine probes as well as corresponding Michael acceptor probes was designed. To maximize enzyme coverage, the probes were designed to vary in their substitution pattern at the C2 atom of the central three-membered heterocycle or the corresponding carbon atom of the Michael acceptor compounds by small (ethyl or allyl) and long (octyl) aliphatic residues as well as by an aromatic residue (benzyl), which enhances the chance of binding into structurally diverse enzyme active sites.

The second feature of these probes is an alkyne handle in their C3 position. The modification of the alkyne tag via the 1,3-dipolar Huisgen cycloaddition (click chemistry, CC) allows to append a fluorophore reporter group for target enzyme visualization via SDS-gel electrophoresis after proteome labeling (Sieber, S.A. et al., Nat Chem Biol, 2006, 2(5): 274-281), as also reported by Cravatt and coworkers. (Evans, M. J.; Cravatt, B. F., Chem Rev 2006, 106, 3279.; Cravatt, B. F.; Wright, A. T.; Kozarich, J. W., Annu. Rev. Biochem., 2008, 77, 383.)

The following compounds were synthesized and subsequently tested by proteome profiling:

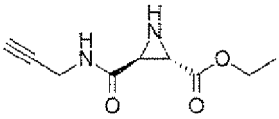

AziEt

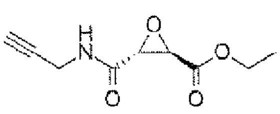

OxyEt

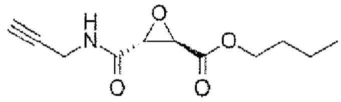

OxyBut

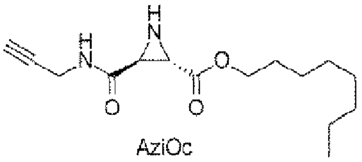

AziOc

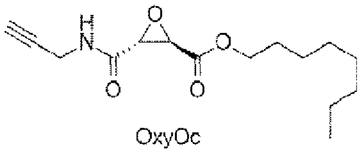

OxyOc

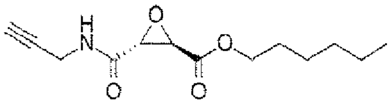

OxyHex

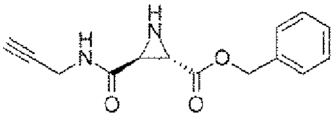

AziBenz

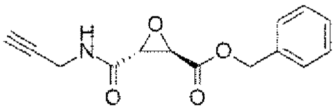

OxyBenz

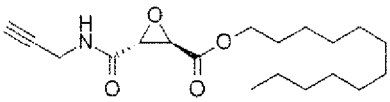

OxyDodec

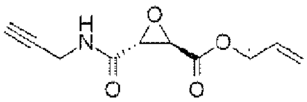

OxyAll

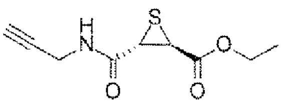

ThiEt

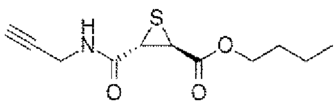

ThiBut

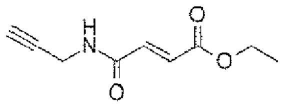

MichEt

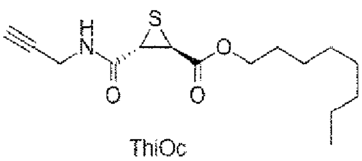

ThiOc

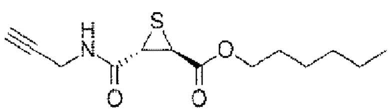

ThiHex

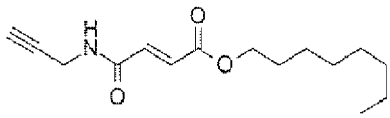

MichOc

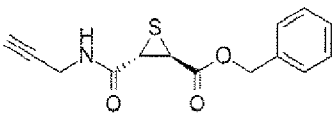

ThiBenz

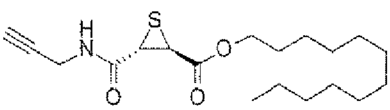

ThiDodec

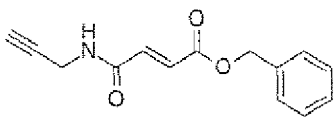

MichBenz

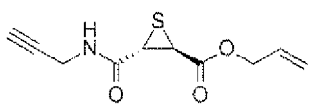

ThiAll

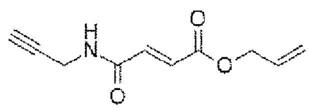

MichAll

The compounds shown above were synthesized in accordance with the following synthetic routes indicated in Schemes 1 and 2:

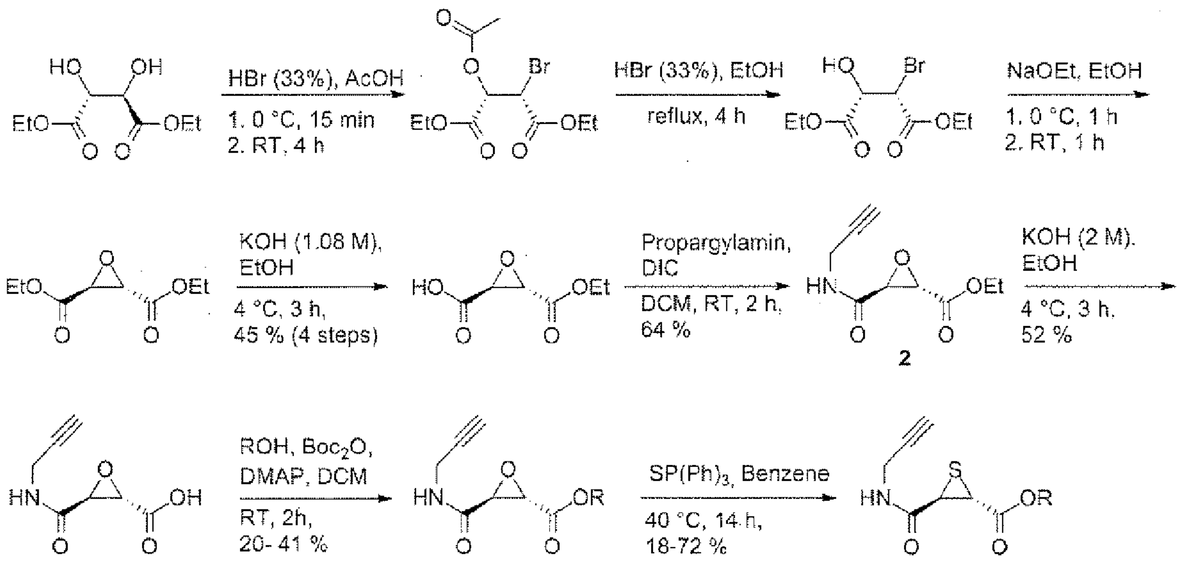


2

**Scheme 1**: Synthesis of thiirane and oxirane compounds. AcOH: acetic acid; EtOH: ethanol; NaOEt: sodium ethoxide; DIC: N,N'-diisopropylcarbodiimide; DCM: dichloromethane; DMAP: 4-dimethylaminopyridine; Ph: phenyl; RT: room temperature.

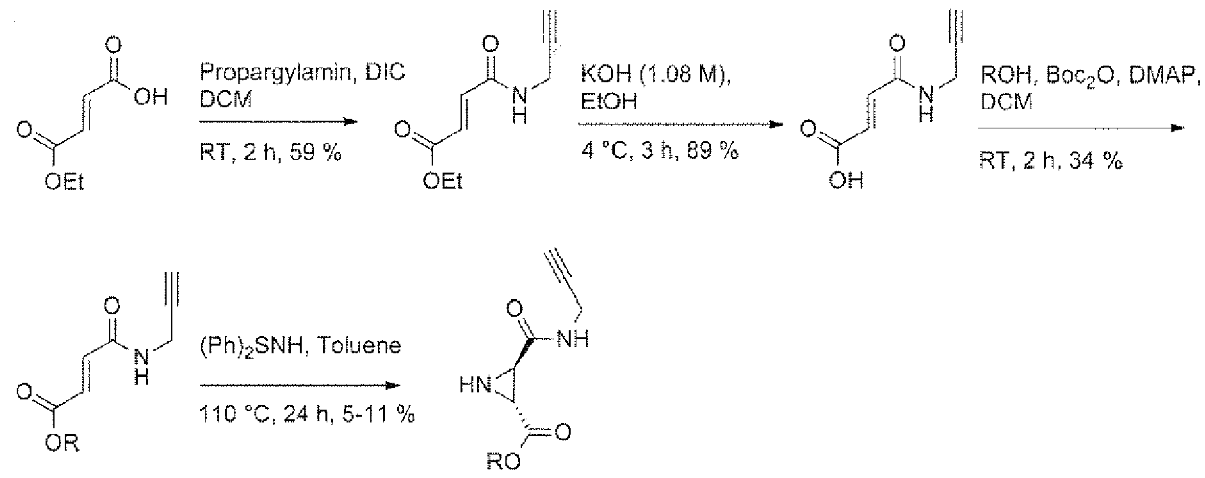


**Scheme 2**: Synthesis of Michael acceptor compounds and aziridine compounds. EtOH: ethanol; DIC: N,N'-diisopropylcarbodiimide; DCM: dichloromethane; DMAP: 4-dimethylaminopyridine; Boc: tert-butoxycarbonyl; $Boc_2O$: di-tert-butyl dicarbonate; Ph: phenyl; RT: room temperature.

The synthesis of the compounds shown above is described in more detail in the following.

**Ethyl (2*R*,3*R*)-oxirane-2,3-dicarboxylate**

(+)-Diethyl L-tartrate (52,0 g, 252.5 mmol) was converted to diethyl (2*S*,3*R*) 2-bromo-3-hydroxy-butanedioate by the method of *Mori.* (Mori, K., Iwasawa, H. Tetrahedron 1980, 87-90.)

The bromohydrin was converted to diethyl (2*R*,3*R*) oxirane-2,3-dicarboxylate by treatment with NaOEt. (Meara, J. P.; Rich, D. H. J Med Chem 1996, 39 ,6, 3357-3366)

The diester was converted into the monoester by addition of 1.08 M KOH in abs. EtOH, which yielded 18.2 g product (yield 45 %, over 4 steps) as a white solid.

$^1H$ NMR (300 MHz, $CDCl_3$) δ 4.34—4.16 (m, 2H), 3.67 (s, 2H), 1.30 (t, *J*=7.2 Hz, 3H); $^{13}C$ NMR (75 MHz, $CDCl_3$) δ 170.62, 166.84, 62.54, 52.16, 51.65, 13.96. ESI - MS (m/z): 178.0712 [M + $NH_4^+$] calc. 178.07099, 338.1083 [2M + $NH_4^+$] calc. 338.1081

**Ethyl (2*R*,3*R*) 3-(*N*-propargylaminocarbonyl)oxirane-2-carboxylate (OxyEt)**

The monoester Ethyl (2R,3R)-oxirane-2,3-dicarboxylate (14.00 g, 87.4 mmol, 1.00 eq) was dissolved in dry dichlormethane (DCM) (500 mL). To this solution *N,N'*-Diisopropylcarbodiimide (DIC) (19.82 g, 96.2 mmol, 1.10 eq) was added. The solution was stirred for 10 min at room temperature to form the reactive ester. Finally, propargylamine (5.06 g, 91.8 mmol, 1.05 eq) was added and stirred at ambient temperature for 15 min. While the solution turned brown, a white precipitate formed. The progress of the reaction was monitored by TLC (EtOAc/*i*-hexane, 1:1, $R_f$: 0.5). After completion of the reaction the solvent was evaporated and the crude was purified by flash-chromatography (hexane/ EtOAc, 2:1, $R_f$: 0.3), which gave 11.00 g (64 %) of a slightly yellow solid.

$^1H$ NMR (300 MHz, $CDCl_3$) δ = 6.36 (s, 1H), 4.27 (qd, *J*=7.1, 1.6 Hz, 2H), 4.06 (ddd, *J*=5.5, 2.6, 0.8 Hz, 2H), 3.72 (d, *J*=1.9 Hz, 1H), 3.53 (d, *J*=1.9 Hz, 1H), 2.27 (t, *J*=2.6 Hz, 1H), 1.32 (t, *J*=7.1 Hz, 3H).

$^{13}C$ NMR (75 MHz, $CDCl_3$) δ = 166.37, 165.69, 78.40, 72.22, 62.38, 53.78, 52.83, 28.81, 14.02.

ESI - MS (m/z): 198.0763 [M + $H^+$] calc. 198.0760, 239.1030 [M + $CH_3CN$ + $H^+$] calc. 239.1026

$[\alpha]_{598}^{25}$ = - 20.1 ($c$ = 1.01, $CHCl_3$)

**(2*R*,3*R*) 3-(*N*-propargylaminocarbonyl)oxirane-2-carboxylic acid**

Ethyl (2R,3R) 3-(N-propargylaminocarbonyl)oxirane-2-carboxylate (11.00 g, 55.78 mmol, 1.00 eq) was dissolved in 100 mL of dry EtOH under Ar atmosphere and cooled to 0 °C. 50 mL of an ethanolic solution of KOH (3.44 g, 61.36 mmol, 1.1 eq) were added slowly while the solution thickened. The solution was stirred for 30 min at 0 °C and 1 h at ambient temperature. The progress of the reaction was monitored by TLC (hexane/EtOAc, 1:1, $R_f$: 0). The solvent was evaporated under reduced pressure and a yellow solid dissolved in water. The solution was washed with DCM. The pH of the solution was adjusted to ~2 by addition of 2 N HCl and three times extracted with EtOAc. The combined organic phases were dried over $Na_2SO_4$, filtered and concentrated *in vacuo* to give 4.90 g (52 %) of a white solid which was used without further purification.
ESI - MS (m/z): 170.0451 [M + $H^+$] calc. 170.0447.

**Butyl (2*R*,3*R*) 3-(*N*-propargylaminocarbonyl)oxirane-2-carboxylate (OxyBut)**

(2R,3R) 3-(N-propargylaminocarbonyl)oxirane-2-carboxylic acid (1.00 g, 5.9 mmol, 1.00 eq) was dissolved in dry Dimethyl sulfoxide (DMSO) (2.5 mL). To this solution, 4-Dimethylaminopyridine (DMAP) (72 mg, 0.6 mmol, 0.10 eq) and *n*-Butanol (482 mg, 6.5 mmol, 1.10 eq) were added. The solution was stirred for 10 min at room temperature until everything was dissolved. Finally, $Boc_2O$ (1.68 g, 7.7 mmol, 1.30 eq) and $Et_3N$ (1.19 g, 11.8 mmol, 2.00 eq) were added. Gas development could be observed while the solution turned brown. The solution was stirred for 2 h at ambient temperature. The progress of the reaction was monitored by TLC. After completion of the reaction EtOAc was added and the mixture was washed with $H_2O$ three times. The aqueous phase was extracted with EtOAc once and the combined organic phases dried over $Na_2SO_4$. The solvent was evaporated and the crude extract purified by flash-chromatography (hexane/EtOAc, 2:1) to give 406 mg of a white solid (31 %).
TLC: $R_f$: 0.5 (hexane/EtOAc, 1:1).
$^1H$ NMR (250 MHz, $CDCl_3$) $\delta$ = 6.27 (s, 1H), 4.30 — 4.12 (m, 2H), 4.06 (ddd, *J*=5.5, 2.6, 1.0 Hz, 2H), 3.73 - 3.68 (m, 1H), 3.52 - 3.51 (m, 1H), 2.26 (t, *J*=2.6 Hz, 1H), 1.74 — 1.56 (m, 2H), 1.47 — 1.30 (m, 2H), 0.94 (t, *J*=7.3 Hz, 3H).
$^{13}C$ NMR (63 MHz, $CDCl_3$) $\delta$ = 166.38, 165.64, 78.33, 72.24, 66.18, 53.77, 52.84, 30.36, 28.79, 18.94, 13.56.
ESI - MS (m/z): 226.1077 [M + $H^+$] calc. 226.1073, 267.1342 [M + $CH_3CN$ + $H^+$] calc. 267.1339.

$[\alpha]^{25}_{598}$ = - 27.5 ($c$ = 1.01, $CHCl_3$)

**Hexyl (2*R*,3*R*) 3-(*N*-propargylaminocarbonyl)oxirane-2-carboxylate (OxyHex)**

(2R,3R) 3-(N-propargylaminocarbonyl)oxirane-2-carboxylic acid (1.00 g, 5.9 mmol, 1.00 eq) was dissolved in dry DMSO (2.5 mL). To this solution, DMAP (72 mg, 0.6 mmol, 0.10 eq) and *n*-Hexanol (664.5 mg, 6.5 mmol, 1.10 eq) were added. The solution was stirred for 10 min at room temperature until everything was dissolved. Finally, $Boc_2O$ (1.68 g, 7.69 mmol, 1.30 eq) and $Et_3N$ (1.19g, 11.8 mmol, 2.00 eq) were added. Gas development could be observed while the solution turned brown. The solution was stirred for 2 h at ambient temperature. The progress of the reaction was monitored by TLC. After completion of the reaction EtOAc was added and the mixture was washed with $H_2O$ three times. The aqueous phase was extracted with EtOAc one time and the combined organic phases dried over $Na_2SO_4$. The solvent was evaporated and the crude extract purified by flash-chromatography (hexane/EtOAc, 3:1) to give 324 mg of a white solid (23 %).
TLC: $R_f$: 0.5 (hexane/EtOAc, 2:1).
$^1H$ NMR (250 MHz, $CDCl_3$) $\delta$ = 6.25 (s, 1H), 4.30 — 4.12 (m, 2H), 4.06 (ddd, *J*=5.4, 2.6, 1.1 Hz, 2H), 3.72 — 3.70 (m, 1H), 3.53 - 3.50 (m, 1H), 2.26 (t, J=2.6 Hz, 1H), 1.76 — 1.60 (m, 2H), 1.45 - 1.22 (m, 6H), 0.97 — 0.82 (m, 3H).
$^{13}C$ NMR (63 MHz, $CDCl_3$) $\delta$ = 166.37, 165.64, 78.31, 72.26, 66.50, 53.78, 52.86, 31.29, 28.79, 28.32, 25.37, 22.45, 13.92.
ESI - MS (m/z): 254.1391 [M + $H^+$] calc. 254.1386, 295.1657 [M + $CH_3CN$ + $H^+$] calc. 295.1652

$[\alpha]^{25}_{598}$ = - 45.0 ($c$ = 1.01, $CHCl_3$)

**Octyl (2*R*,3*R*) 3-(*N*-propargylaminocarbonyl)oxirane-2-carboxylate (OxyOc)**

(2R,3R) 3-(N-propargylaminocarbonyl)oxirane-2-carboxylic acid (400 mg, 2.36 mmol, 1.00 eq) was dissolved in dry DMSO (1 mL). To this solution, DMAP (29 mg, 0.24 mmol, 0.10 eq) and *n*-Octanol (339 mg, 2.60 mmol, 1.10 eq) were added. The solution was stirred for 10 min at room temperature until everything was dissolved. Finally, $Boc_2O$ (671

mg, 3.07 mmol, 1.30 eq) and $Et_3N$ (479 mg, 4.73 mmol, 2.00 eq) were added. Gas development could be observed while the solution turned brown. The solution was stirred for 2 h at ambient temperature. The progress of the reaction was monitored by TLC. After completion of the reaction EtOAc was added and the mixture was washed with $H_2O$ three times. The aqueous phase was extracted with EtOAc once and the combined organic phases dried over $Na_2SO_4$. The solvent was evaporated and the crude extract purified by flash-chromatography (hexane/EtOAc, 4:1) to give 275 mg of a white solid (41 %).

TLC: $R_f$: 0.27 (hexane/EtOAc, 3:1).

$^1$H NMR (300 MHz, $CDCl_3$) $\delta$ = 6.31 (s, 1H), 4.30 - 4.12 (m, 2H), 4.07 (ddd, *J*=5.4, 2.5, 1.2 Hz, 2H), 3.72 (d, *J*=1.9 Hz, 1H), 3.53 (d, *J*=1.9 Hz, 1H), 2.27 (t, *J*=2.6 Hz, 1H), 1.75 - 1.61 (m, 2H), 1.45 - 1.17 (m, 10H), 0.90 (t, *J*=6.7 Hz, 3H).

$^{13}$C NMR (75 MHz, $CDCl_3$) $\delta$ = 166.43, 165.68, 78.37, 72.25, 66.52, 53.79, 52.86, 31.73, 29.10, 28.81, 28.38, 25.73, 22.60, 14.05.

ESI - MS (m/z): 282.1698 [M + H$^+$] calc. 282.1699, 299.1963 [M + $NH_4^+$] calc. 299.1965, 280.1559 [M - H$^+$] calc. 280.1554

$[\alpha]_{598}^{25}$ = - 62.1 (*c* = 1.01, $CHCl_3$)

**Dodecyl (2*R*,3*R*) 3-(*N*-propargylaminocarbonyl)oxirane-2-carboxylate (OxyDodec)**

(2R,3R) 3-(N-propargylaminocarbonyl)oxirane-2-carboxylic acid (1.00 g, 5.9 mmol, 1.00 eq) was dissolved in dry DMSO (2.5 mL). To this solution, DMAP (72 mg, 0.6 mmol, 0.10 eq) and n-dodecanol (1.21 g, 6.5 mmol, 1,10 eq) were added. The solution was stirred for 10 min at room temperature until everything was dissolved. Finally, $Boc_2O$ (1.68 g, 7.7 mmol, 1.30 eq) and $Et_3N$ (1.19 g, 11.8 mmol, 2.00 eq) were added. Gas development could be observed while the solution turned brown. The solution was stirred for 2 h at ambient temperature. The progress of the reaction was monitored by TLC. After completion of the reaction EtOAc was added and the mixture was washed with $H_2O$ three times. The aqueous phase was extracted with EtOAc once and the combined organic phases dried over $Na_2SO_4$. The solvent was evaporated and the crude extract purified by flash-chromatography (hexane/EtOAc, 10:1) to give 517 mg of a white solid (26 %).

TLC: $R_f$: 0.5 (hexane/EtOAc, 2:1).

$^1$H NMR (250 MHz, $CDCl_3$) $\delta$ = 6.26 (s, 1H), 4.28 - 4.10 (m, 2H), 4.06 (ddd, *J*=5.4, 2.5, 0.9 Hz, 2H), 3.72 - 3.70 (m, 1H), 3.53 — 3.50 (m, 1H), 2.26 (t, *J*=2.6 Hz, 1H), 1.74 — 1.56 (m, 2H), 1.43 - 1.19 (m, 18H), 0.88 (t, *J*=6.6 Hz, 3H).

$^{13}$C NMR (63 MHz, $CDCl_3$) $\delta$ = 166.37, 165.64, 78.32, 72.25, 66.50, 53.77, 52.86, 31.87, 29.58, 29.51, 29.43, 29.30, 29.13, 28.79, 28.37, 25.71, 22.65, 14.07.

ESI - MS (m/z): 338.2330 [M + H$^+$] calc. 338.2325.

$[\alpha]_{598}^{25}$ = - 38.2 (*c* = 1.01, $CHCl_3$)

**Benzyl (2*R*,3*R*) 3-(*N*-propargylaminocarbonyl)oxirane-2-carboxylate (OxyBenz)**

(2R,3R) 3-(N-propargylaminocarbonyl)oxirane-2-carboxylic acid (400 mg, 2.36 mmol, 1.00 eq) was dissolved in dry DMSO (1 mL). To this solution, DMAP (29 mg, 0.24 mmol, 0.10 eq) and benzyl alcohol (282 mg, 2.60 mmol, 1.10 eq) were added. The solution was stirred for 10 min at room temperature until everything was dissolved. Finally, $Boc_2O$ (671 mg, 3.07 mmol, 1.30 eq) and $Et_3N$ (479 mg, 4.73 mmol, 2.00 eq) were added. Gas development could be observed while the solution turned brown. The solution was stirred for 2 h at ambient temperature. The progress of the reaction was monitored by TLC. After completion of the reaction EtOAc was added and the mixture was washed with $H_2O$ three times. The aqueous phase was extracted with EtOAc once and the combined organic phases dried over $Na_2SO_4$. The solvent was evaporated and the crude extract purified by flash-chromatography (hexane/EtOAc, 3:1) to give 120 mg of a white solid (19.6 %).

TLC: $R_f$: 0.3 (hexane/EtOAc, 2:1) $^1$H NMR (300 MHz, $CDCl_3$) $\delta$ = 7.41 - 7.35 (m, 5H), 6.28 (s, 1H), 5.24 (q, *J*=12.1 Hz, 2H), 4.06 (ddd, *J*=5.4, 2.5, 1.8 Hz, 2H), 3.75 (d, *J*=1.9 Hz, 1H), 3.57 (d, *J*=1.9 Hz, 1H), 2.27 (t, *J*=2,6 Hz, 1H).

$^{13}$C NMR (75 MHz, $CDCl_3$) $\delta$ = 166.23, 165.52, 134.44, 128.85, 128.74, 128.63, 78.32, 72.29, 68.03, 53.84, 52.82, 28.82.

ESI - MS (m/z): 260.0916 [M + H$^+$] calc. 250.0917, 277.1181 [M + $NH_4^+$] calc. 277.1183, 258.0781 [M - H$^+$] calc. 258.0772

$[\alpha]_{598}^{25}$ = - 48.2 (*c* = 1.01, $CHCl_3$)

**Allyl (2*R*,3*R*) 3-(N-propargylaminocarbonyl)oxirane-2-carboxylate (OxyAll)**

(2R,3R) 3-(N-propargylaminocarbonyl)oxirane-2-carboxylic acid (400 mg, 2.36 mmol, 1.00 eq) was dissolved in dry DMSO (1 mL). To this solution, DMAP (29 mg, 0.24 mmol, 0.10 eq) and allyl alcohol (151 mg, 2.60 mmol, 1.10 eq) were added. The solution was stirred for 10 min at room temperature until everything was dissolved. Finally, $Boc_2O$ (671 mg, 3.07 mmol, 1.30 eq) and $Et_3N$ (479 mg, 4.73 mmol, 2.00 eq) were added. Gas development could be observed while the solution turned brown. The solution was stirred for 2 h at ambient temperature. The progress of the reaction was monitored by TLC. After completion of the reaction EtOAc was added and the mixture was washed with $H_2O$ three times. The aqueous phase was extracted with EtOAc once and the combined organic phases dried over $Na_2SO_4$. The solvent was evaporated and the crude extract purified by flash-chromatography (hexane/EtOAc, 3:1) to give 111 mg of a slightly red solid (22.4 %).

TLC: $R_f$: 0.5 (hexane/EtOAc, 1:1)

$^1$H NMR (599 MHz, $CDCl_3$) $\delta$ = 5.37 (dd, *J*=2.8, 1.4 Hz, 1H), 5.34 (dd, *J*=2.8, 1.5 Hz, 1H), 5.31 (dd, *J*=1.1 Hz, 1H), 5.29 (dd, *J*=1.1 Hz, 1H), 4.72 - 4.64 (m, 2H), 4.09 - 4.00 (m, 2H), 3.72 (d, *J*=1.9 Hz, 1H), 3.53 (d, *J*=1.9 Hz, 1H), 2.25 (t, *J*=2.6 Hz, 1H).

13C NMR (151 MHz, $CDCl_3$) $\delta$ = 166.01, 165.50, 130.74, 119.84, 78.27, 72.30, 66.79, 53.80, 52.76, 28.80.

ESI - MS (m/z): 208.0619 [M - H$^+$] calc. 208.061524.

$[\alpha]_{598}^{25}$ = - 35.0 ($c$ = 1.01, $CHCl_3$)

**General procedure for the preparation of the thiirane probes:**

All thiiranes were synthesized based on the procedure published by *Korn.* (Korn, A. C., Moroder L. J Chem Res (S) 1995, 102-103.)

**Ethyl (2*S*,3*S*) 3-(*N*-propargylaminocarbonyl)thiirane-2-carboxylate (ThiEt)**

Ethyl (2R,3R) 3-(N-propargylaminocarbonyl)oxirane-2-carboxylate (200 mg, 1.01 mmol, 1.00 eq) was suspended in dry Toluene (1.5 mL). To this solution, triphenylphosphine sulfide (597 mg, 2.02 mmol, 2.00 eq) and TFA (116 mg, 1.01 mmol, 1.00 eq) were added. The solution was stirred for 6 h at 50 °C. The progress of the reaction was monitored by TLC ($HCCl_3$ and then EtOAc/*i*-hexane, 2:1). The solvent was evaporated under reduced pressure and the yellow solid dissolved in chloroform. The crude was directly applied onto a silica column and was purified by flash-chromatography ($HCCl_3$ until elution of the first spot, then EtOAc/*i*-hexane, 4:1) to give 84 mg of a white crystalline solid (39 %).

TLC: $R_f$: 0.5 (hexane/EtOAc, 2: 1)

$^1$H NMR (250 MHz, $CDCl_3$) $\delta$ = 6.30 (s, 1H), 4.23 (q, *J*=7.2 Hz, 2H), 4.09 - 4.03 (m, 2H), 3.75 (d, *J*=4.3 Hz, 1H), 3.59 (d, *J*=4.3 Hz, 1H), 2.27 (t, *J*=2.6 Hz, 1H), 1.30 (t, *J*=7.1 Hz, 3H).

$^{13}$C NMR (63 MHz, $CDCl_3$) $\delta$ = 168.32, 166.13, 78.49, 72.23, 62.42, 36.47, 34.21, 29.68, 13.98.

ESI - MS (m/z): 214.0535 [M + H$^+$] calc. 214.0532, 255.0802 [M + $CH_3CN$ + H$^+$] calc. 255.0797.

$[\alpha]_{598}^{25}$ = + 128.4 ($c$ = 1.01, $CHCl_3$)

**Butyl (2*S*,3*S*) 3-(*N*-propargylaminocarbonyl)thiirane-2-carboxylate (ThiBut)**

Butyl (2*R*,3*R*) 3-(*N*-propargylaminocarbonyl)oxirane-2-carboxylate (225 mg, 1.0 mmol, 1.0 eq) was suspended in dry toluene (1.0 mL). To this solution, triphenylphosphine sulfide (589 mg, 2.0 mmol, 2.0 eq) and TFA (114 mg, 1.0 mmol, 1.0 eq) were added. The solution was stirred for 6 h at 50 °C. The solvent was evaporated under reduced pressure and the yellow solid dissolved in chloroform. The crude was directly applied onto a silica column and was purified by flash-chromatography ($HCCl_3$ until elution of the first spot, then EtOAc/*i*-hexane, 3:1) to give 84 mg of a white crystalline solid (18.2 %).

TLC: $R_f$: 0.3 (hexane/EtOAc, 2:1).

$^1$H NMR (250 MHz, $CDCl_3$) $\delta$ = 6.30 (s, 1H), 4.20 (t, *J*=6.6 Hz, 2H), 4.13 - 4.03 (m, 2H), 3.77 (d, *J*=4.3 Hz, 1H), 3.61 (d, *J*=4.3 Hz, 1H), 2.29 (t, *J*=2.6 Hz, 1H), 1.76 - 1.61 (m, 2H), 1.51 - 1.30 (m, 2H), 0.97 (t, *J*=7.3 Hz, 3H).

$^{13}C$ NMR (63 MHz, $CDCl_3$) $\delta$ = 168.55, 166.81, 99.94, 77.20, 72.28, 66.30, 36.50, 34.22, 30.54, 18.97, 13.50.
ESI - MS (m/z): 242.0849 [M + $H^+$] calc. 242.0845, 283.1116 [M + $CH_3CN$ + $H^+$] calc. 283.1111.

$[\alpha]^{25}_{598}$ = + 100.8 (*c* = 1.01, $CHCl_3$)

**Hexyl (2*S*,3*S*) 3-(*N*-propargylaminocarbonyl)thiirane-2-carboxylate (ThiHex)**

Hexyl (2*R*,3*R*) 3-(*N*-propargylaminocarbonyl)oxirane-2-carboxylate (253 mg, 1.0 mmol, 1.0 eq) was suspended in dry Toluene (1.0 mL). To this solution, triphenylphosphine sulfide (589 mg, 2.0 mmol, 2.00 eq) and TFA (270 mg, 2.4 mmol, 2.4 eq) were added. The solution was stirred for 6 h at 50 °C. The progress of the reaction was monitored by TLC ($HCCl_3$ and then EtOAc/*i*-hexane, 2:1). The solvent was evaporated under reduced pressure and the yellow solid dissolved in chloroform. The crude was directly applied onto a silica column and was purified by flash-chromatography ($HCCl_3$ until elution of the first spot, then EtOAc/*i*-hexane, 3:1) to give 160 mg of a white crystalline solid (59.4 %).
TLC: $R_f$: 0.3 (hexane/EtOAc, 2:1).
$^1H$ NMR (250 MHz, $CDCl_3$) $\delta$ = 6.35 (s, 1H), 4.23 - 4.15 (m, 2H), 4.12 - 4.05 (m, 2H), 3.78 (d, *J*=4.3 Hz, 1H), 3.62 (d, *J*=4.3 Hz, 1H), 2.30 (t, *J*=2.6 Hz, 1H), 1.77 - 1.60 (m, 2H), 1.45 - 1.25 (m, 6H), 0.93 (t, *J*=6.7 Hz, 3H).
$^{13}C$ NMR (63 MHz, $CDCl_3$) $\delta$ = 168.45, 166.28, 78.45, 72.29, 66.61, 36.49, 34.26, 31.34, 29.71, 28.36, 25.43, 22.48, 13.96.
ESI - MS (m/z): 270.1162 [M + $H^+$] calc. 270.1158, 311.1430 [M + $CH_3CN$ + $H^+$] calc. 311.1424.

$[\alpha]^{25}_{598}$ = + 93.3 (*c* = 1.01, $CHCl_3$)

**Octyl (2*S*,3*S*) 3-(*N*-propargylaminocarbonyl)thiirane-2-carboxylate (ThiOc)**

Octyl (2*R*,3*R*) 3-(*N*-propargylaminocarbonyl)oxirane-2-carboxylate (214 mg, 760 μmol, 1.0 eq) was suspended in dry toluene (1.0 mL). To this solution, triphenylphosphine sulfide (448 mg, 1.52 mmol, 2.0 eq) and TFA (86.72 mg, 760 μmol, 1.0 eq) were added. The solution was stirred for 6 h at 50 °C. The solvent was evaporated under reduced pressure and the yellow solid dissolved in chloroform. The crude was directly applied onto a silica column and was purified by flash-chromatography ($HCCl_3$ until elution of the first spot, then EtOAc/*i*-hexane, 3:1) to give 89 mg of a white crystalline solid (39,4 %).
TLC: $R_f$: 0.3 (hexane/EtOAc, 3:1).
$^1H$ NMR (300 MHz, $CDCl_3$) $\delta$ = 6.77 (s, 1H), 4.17 (t, *J*=6.7 Hz, 2H), 4.07 (ddd, J=5.3, 4.2, 2.6 Hz, 2H), 3.80 (d, *J*=4.2 Hz, 1H), 3.65 (d, *J*=4.2 Hz, 1H), 2.28 (t, *J*=2.6 Hz, 1H), 1.72 — 1.60 (m, 2H), 1.40 — 1.22 (m, 10H), 0.89 (t, *J*=6.7 Hz, 3H).
$^{13}C$ NMR (75 MHz, $CDCl_3$) $\delta$ = 168.79, 167.09, 78.29, 72.36, 66.74, 60.70, 36.25, 34.14, 31.73, 29.88, 29.11, 28.36, 25.74, 22.60, 14.04.
ESI - MS (m/z): 298.1468 [M + $H^+$] calc. 298.1471, 595.2852 [2M + $H^+$] calc. 595.2870.

$[\alpha]^{25}_{598}$ = + 111.8 (*c* = 1.01, $CHCl_3$)

**Dodecyl (2*S*,3*S*) 3-(*N*-propargylaminocarbonyl)thiirane-2-carboxylate (ThiDodec)**

Dodecyl (2*R*,3*R*) 3-(*N*-propargylaminocarbonyl)oxirane-2-carboxylate (337 mg, 760 μmol, 1.00 eq) was suspended in dry Toluene (1.0 mL). To this solution, triphenylphosphine sulfide (558 mg, 2.00 mmol, 2.00 eq) and TFA (600 mg, 5.2 mmol, 6.85 eq) were added. The solution was stirred for 6 h at 50 °C. The solvent was evaporated under reduced pressure and the yellow solid dissolved in chloroform. The crude was directly applied onto a silica column and was purified by Flash-Chromatography ($HCCl_3$ until elution of the first spot, then EtOAc/*i*-hexane, 3:1) to give 178 mg of a white crystalline solid (50.3 %).
TLC: $R_f$: 0.3 (hexane/EtOAc, 2:1).
$^1H$ NMR (250 MHz, $CDCl_3$) $\delta$ = 6.29 (s, 1H), 4.18 (t, *J*=6.7 Hz, 2H), 4.11 - 4.06 (m, 2H), 3.77 (d, *J*=4.3 Hz, 1H), 3.61 (d, *J*=4.3 Hz, 1H), 2.29 (t, *J*=2.6 Hz, 1H), 1.75 - 1.60 (m, 4H), 1.41 - 1.21 (m, 16H), 0.91 (t, *J*=6.6 Hz, 3H).
$^{13}C$ NMR (63 MHz, $CDCl_3$) $\delta$= 168.46, 166.27, 78.53, 72.27, 66.62, 36.50, 34.26, 31.91, 29.70, 29.63, 29.55, 29.47, 29.34, 29.18, 28.41, 25.77, 22.68, 14.11.
ESI - MS (m/z): 354.2101 [M + $H^+$] calc. 354.2097.

$[\alpha]^{25}_{598}$ = + 91.8 (*c* = 1.01, $CHCl_3$)

**Benzyl (2*S*,3*S*) 3-(*N*-propargylaminocarbonyl)thiirane-2-carboxylate (ThiBenz)**

Benzyl (2*R*,3*R*) 3-(*N*-propargylaminocarbonyl)oxirane-2-carboxylate (337 mg, 760 μmol, 1.00 eq) was suspended in dry Toluene (1.0 mL). To this solution, triphenylphosphine sulfide (558 mg, 2.0 mmol, 2.00 eq) and TFA (600 mg, 5.2 mmol, 6.85 eq) were added. The solution was stirred for 6 h at 50 °C. The solvent was evaporated under reduced pressure and the yellow solid dissolved in chloroform. The solved crude was applied directly onto a silica column and was purified by flash-chromatography ($HCCl_3$ until elution of the first spot, then EtOAc/*i*-hexane, 4: 1) to give 77 mg of a white crystalline solid (72.4 %).

TLC: $R_f$: 0.5 (hexane/EtOAc, 3:1).

$^1H$ NMR (300 MHz, $CDCl_3$) δ 7.46 — 7.33 (m, 5H), 6.34 (s, 1H), 5.28 - 5.15 (m, 2H), 4.06 (dt, *J*=5.4, 2.7 Hz, 2H), 3.79 (dd, *J*=4.2, 0.4 Hz, 1H), 3.67 (dd, *J*=4.2, 0.5 Hz, 1H), 2.27 (td, *J*=2.6, 0.4 Hz, 1H).

$^{13}C$ NMR (75 MHz, $CDCl_3$) δ 168.36, 166.10, 134.77, 128.70, 128.44, 78.51, 72.28, 68.09, 36.50,34.11,29.72.

ESI - MS (m/z): 293.0952 [M + $NH_4^+$] calc. 293.0954, 551.1259 [2M + $H^+$] calc. 551.1304, 274.0553 [2M - $H^+$] calc. 274.0543

$[\alpha]_{598}^{25} = +94.6$ ($c$ = 1.01, $CHCl_3$)

**Allyl (2*S*,3*S*) 3-(*N*-propargylaminocarbonyl)thiirane-2-carboxylate (ThiAll)**

Allyl (2*R*,3*R*) 3-(N-propargylaminocarbonyl)oxirane-2-carboxylate (100 mg, 478 μmol, 1.0 eq) was suspended in dry Toluene (1.0 mL). To this solution, triphenylphosphine sulfide (281 mg, 956 μmol, 2.0 eq) and TFA (54.5 mg, 478 μmol, 1.0 eq) were added. The solution was stirred for 6 h at 50 °C. The solvent was evaporated under reduced pressure and the yellow solid dissolved in chloroform. The crude was directly applied onto a silica column and was purified by flash-chromatography ($HCCl_3$ until elution of the first spot, then EtOAc/*i*-hexane, 2:1) to give 66 mg of a white crystalline solid (61 %).

TLC: $R_f$: 0.45 (hexane/EtOAc, 2:1).

$^1H$ NMR (300 MHz, $CDCl_3$) δ = 6.70 (s, 1H), 6.00 - 5.82 (m, 1H), 5.41 - 5.38 (m, 1 H), 5.38 - 5.33 (m, 1H), 5.32 (dt, *J*=2.2, 0.7 Hz, 1H), 5.28 (dt, *J*=2.2, 0.7 Hz, 1H), 4.79 - 4.54 (m, 2H), 3.81 (d, *J*=4.2 Hz, 1H), 3.68 (d, *J*=4.2 Hz, 1H), 2.29 (t, *J*=2.7 Hz, 1H).

$^{13}C$ NMR (75 MHz, $CDCl_3$) δ = 168.35, 166.87, 131.01, 119.41, 78.30, 72.40, 66.94, 36.31, 34.02, 29.88.

ESI - MS (m/z): 226.0536 [M + $H^+$] calc. 226.0532, 267.0803 [M + $CH_3CN$ + $H^+$] calc. 267.0798.

$[\alpha]_{598}^{25} = +95.7$ ($c$ = 1.01, $CHCl_3$)

**(*E*)-ethyl 4-oxo-4-(prop-2-yn-1-ylamino)but-2-enoate (MichEt)**

Fumaric acid monoethylester (10.0 g, 69.4 mmol, 2.00 eq) was solved in dry DCM (150 mL). To this solution, DCC (14.3 g, 69.4 mmol, 2.00 eq) and DIPEA (4.5 g, 34.7 mmol, 1.00 eq) were added. The solution was incubated for 10 min at room temperature to form the reactive ester. Finally, propargylamine (1.9 g, 55.08 mmol, 1.00 eq) was added and stirred at ambient temperature for 15 min. While the solution turned brown, a white precipitate formed. After completion of the reaction the solvent was evaporated and the crude was purified by flash-chromatography (hexane/EtOAc, 3:1), which gave 3.7 g (59 %) of a slightly yellow solid.

TLC: $R_f$: 0.3 (hexane/EtOAc, 2:1).

$^1H$ NMR (250 MHz, $CDCl_3$) δ = 7.01 - 6.80 (m, 2H), 6.10 (s, 1H), 4.29 (q, *J*=7.1 Hz, 2H), 4.19 (dd, *J*=5.3, 2.6 Hz, 2H), 2.30 (t, *J*=2.6 Hz, 1H), 1.35 (t, *J*=7.1 Hz, 3H).

$^{13}C$ NMR (63 MHz, $CDCl_3$) δ = 165.36, 163.19, 135.22, 131.31, 78.65, 72.25, 61.30, 29.61, 14.12.

ESI - MS (m/z): 182.0814 [M + $H^+$] calc. 182.0811.

**Ethyl (2*R**,3*R**) 3-(*N*-propargylaminocarbonyl)aziridine-2-carboxylate (AziEt)**

(*E*)-ethyl 4-oxo-4-(prop-2-yn-1-ylamino)but-2-enoate (453 mg, 2.5 mmol, 1.0 eq) was suspended in toluene (10 mL). To this solution *S*,*S*-diphenylsufilimine monohydrate (643 mg, 3.0 mmol, 1.2 eq) was added. The suspension was stirred for 24 h at 110 °C. The solvent

was evaporated under reduced pressure and the yellow solid was purified by flash-chromatography (EtOAc/*i*-hexane, 1:1) to give 55 mg of a yellow oil (11 %).

TLC: $R_f$: 0.3 (hexane/EtOAc, 2:1).

$^1H$ NMR (250 MHz, DMSO) δ = 4.14 (dd, *J*=14.3, 7.2 Hz, 2H), 3.92 (dd, *J*=4.9, 1.8 Hz, 2H), 2.73 (s, 1H), 2.59

(s, 1H), 2.26 - 2.21 (m, 1H), 1.22 (t, *J*=7.1 Hz, 3H).

$^{13}C$ NMR (63 MHz, $CDCl_3$) δ = 170.09, 167.60, 78.87, 71.81, 62.29, 37.36, 35.63, 28.84, 14.07.

ESI - MS (m/z): 197.0923 [M + $H^+$] calc. 197.0920.

**(*E*)-4-oxo-4-(prop-2-yn-1-ylamino)but-2-enoic acid**

(*E*)-ethyl 4-oxo-4-(prop-2-yn-1-ylamino)but-2-enoate (3.70 g, 20.42 mmol, 1.0 eq) was dissolved in 30 mL of dry EtOH under Ar atmosphere and cooled to 0 °C. 50 mL of an ethanolic solution of KOH (1.26 g, 22.46 mmol, 1.1 eq) were added slowly. The solution was stirred for 30 min at 0 °C and 1 h at ambient temperature. The progress of the reaction was monitored by TLC (hexane/EtOAc, 1:1, $R_f$: 0). The solvent was evaporated under reduced pressure and the yellow solid dissolved in water. The solution was washed with DCM. The pH of the solution was adjusted to -2 by addition of 2 N HCl and three times extracted with EtOAc. The combined organic phases were dried over $Na_2SO_4$, filtered and concentrated *in vacuo* to give 2.8 g (89.5 %) of a yellow solid which was used without further purification.

ESI - MS (m/z): 154.0500 [M + $NH_4^+$] calc. 154.0498.

**(*E*)-octyl 4-oxo-4-(prop-2-yn-1-ylamino)but-2-enoate (MichOc)**

(*E*)-4-oxo-4-(prop-2-yn-1-ylamino)but-2-enoic acid (1.00 g, 6.53 mmol, 1.00 eq) was dissolved in dry DMSO (2.0 mL). To this solution, DMAP (79.77 mg, 653 μmol, 0.10 eq) and *n*-Octanol (850.00 mg, 6.53 mmol, 1.00 eq) were added. The solution was stirred for 10 min at room temperature until everything was dissolved. Finally, $Boc_2O$ (1.85 g, 8.49 mmol, 1.30 eq) and $Et_3N$ (1.32 g, 13.05 mmol, 2.00 eq) were added. Gas development could be observed while the solution turned brown. The solution was stirred for 2 h at ambient temperature. The progress of the reaction was monitored by TLC. After completion of the reaction EtOAc was added and the mixture was washed with $H_2O$ three times. The aqueous phase was extracted with EtOAc once and the combined organic phases dried over $Na_2SO_4$. The solvent was evaporated and the crude extract purified by flash-chromatography (hexane/EtOAc, 3:1) to give 584 mg of a yellow oil (34 %).

TLC: $R_f$: 0.5 (hexane/EtOAc, 3:1).

$^1H$ NMR (250 MHz, $CDCl_3$) δ = 6.92 (dd, *J*=15.4 Hz, 2H), 6.41 (s, 1 H), 4.27 - 4.06 (m, 4H), 2.29 (t, *J*=2.6 Hz, 1H), 1.81 - 1.51 (m, 2H), 1.43 - 1.20 (m, 10H), 0.90 (t, *J*=6.6 Hz, 3H).

$^{13}C$ NMR (63 MHz, $CDCl_3$) δ = 165.49, 163.25, 135.27, 131.21, 78.70, 72.14, 65.46, 38.09, 31.72, 29.13, 28.83, 28.49, 25.84, 22.59, 14.03.

ESI - MS (m/z): 206.1755 [M + $H^+$], calc. 266.1750.

**(*E*)-benzyl 4-oxo-4-(prop-2-yn-1-ylamino)but-2-enoate (MichBenz)**

(*E*)-4-oxo-4-(prop-2-yn-1-ylamino)but-2-enoic acid (1.00 g, 6.53mmol, 1.00 eq) was dissolved in dry DMSO (2 mL). To this solution, DMAP (79.77 mg, 653 μmol, 0.10 eq) and benzylic alcohol (850 mg, 6.53 mmol, 1.00 eq) were added. The solution was stirred for 10 min at room temperature until everything was dissolved. Finally, $Boc_2O$ (1.85 g, 8.49 mmol, 1.30 eq) and $Et_3N$ (1.32 g, 13.05 mmol, 2.00 eq) were added. Gas development could be observed while the solution turned brown. The solution was stirred for 2 h at ambient temperature. The progress of the reaction was monitored by TLC. After completion of the reaction EtOAc was added and the mixture was washed with $H_2O$ three times. The aqueous phase was extracted with EtOAc once and the combined organic phases dried over $Na_2SO_4$. The solvent was evaporated and the crude extract purified by flash-chromatography (hexane/EtOAc, 3:1) to give 584 mg of a yellow oil (34 %).

TLC: $R_f$: 0.5 (hexane/EtOAc, 3:1)

$^1H$ NMR (360 MHz, $CDCl_3$) δ = 7.75 (d, *J*=15.4 Hz, 1H), 7.45 - 7.30 (m, 6H), 6.77 (d, *J*=15.4 Hz, 1H), 5.25 (s, 2H), 4.49 (d, *J*=2.4 Hz, 2H), 2.20 (t, *J*=2.4 Hz, 1H).

$^{13}C$ NMR (63 MHz, $CDCl_3$) δ= 167.83, 167.03, 79.10, 71.99, 66.67, 37.57, 35.79, 31.91, 29.30, 28.63, 25.95, 22.78, 14.23.

ESI - MS (m/z): 244.0972 [M + $H^+$] calc. 244.0967, 285,1239 [M + $CH_3CN$ + $H^+$] calc. 285.1233.

**Octyl (2*R**,3*R**) 3-(*N*-propargylaminocarbonyl)aziridine-2-carboxylate (AziOc)**

(*E*)-octyl 4-oxo-4-(prop-2-yn-1-ylamino)but-2-enoate (584 mg, 2.20 mmol, 1.00 eq) was suspended in Toluene (10.0 mL). To this solution *S*,*S*-diphenylsulfilimine monohydrate (565 mg, 2.64 mmol, 1.20 eq) was added. The suspension was stirred for 24 h at 110 °C. The solvent was evaporated under reduced pressure and the yellow solid was purified by flash-chromatography (EtOAc/*i*-hexane, 3:1) to give 29 mg of a yellow oil (5 %).

TLC: $R_f$: 0.2 (EtOAc/ hexane, 3:1)

$^1H$ NMR (250 MHz, $CDCl_3$) $\delta$ = 6.62 (s, 1H), 4.22 - 4.14 (m, 2H), 4.04 (dd, *J*=5.5, 2.5 Hz, 2H), 2.87 (d, *J*=2.0 Hz, 1H), 2.66 (d, *J*=1.9 Hz, 1H), 2.25 (t, *J*=2.5 Hz, 1H), 1.84 (s, 1H), 1.75 - 1.60 (m, 2H), 1.44 - 1.19 (m, 10H), 0.90 (t, *J*=6.4 Hz, 3H).

$^{13}C$ NMR (63 MHz, $CDCl_3$) $\delta$ = 167.83, 167.03, 79.10, 71.99, 66.67, 37.57, 35.79, 31.91, 29.30, 28.63, 26.79, 25.95, 22.78, 14.23.

ESI - MS (m/z): 281.1864 [M + $H^+$] calc. 281.1859.

**Benzyl (2*R**,3*R**) 3-(N-propargylaminocarbonyl)aziridine-2-carboxylate (AziBenz)** (*E*)-benzyl 4-oxo-4-(prop-2-yn-1-ylamino)but-2-enoate (584 mg, 2.20 mmol, 1.0 eq) was suspended in toluene (10 mL). To this solution *S*,*S*-diphenylsulfilimine monohydrate (565 mg, 2.64 mmol, 1.2 eq) was added. The suspension was stirred for 24 h at 110 °C. The solvent was evaporated under reduced pressure and the yellow solid was purified by flash-chromatography (EtOAc/*i*-hexane, 3:1) to give 29 mg of a yellow oil (5 %).

TLC: $R_f$: 0.3 (EtOAc/ hexane, 3:1)

$^1H$ NMR (250 MHz, $CDCl_3$) $\delta$ = 7.69 - 7.60 (m, 2H), 7.47 - 7.34 (m, 2H), 7.14 (s, 1H), 6.52 (s, 1H), 5.19 - 4.94 (m, 2H), 3.94 - 3.75 (m, 2H), 2.78 (d, *J*=8.4 Hz, 1H), 2.55 (d, *J*=6.4 Hz, 1H), 2.10 (t, *J*=2.5 Hz, 1H), 1,69 (s, 1H).

$^{13}C$ NMR (63 MHz, $CDCl_3$) $\delta$ = 168.10, 167.60, 159.66, 135.59, 128.89, 128.70, 78.40, 71.97, 70.84, 38.08, 35.81, 26.41.

ESI - MS (m/z): 259.1082 [M + $H^+$], calc. 259.1076.

<u>Proteome profiling</u>

The above described probe library was screened against living cells of *S. aureus* and *L. welshimeri* under *in situ* conditions. Bacterial cells were grown into stationary phase and subsequently incubated for 1 h with varying amounts of probes. The cells were washed, lyzed and the proteomic extract treated with rhodamine azide under click chemistry (CC) conditions.

The corresponding SDS gels of these initial studies revealed strong labeling of individual proteins with optimal probe concentrations of 200 µM for oxiranes and 20 µM for thiiranes as well as aziridines that were sufficient to achieve saturated labeling of the majority of targets. Interestingly, distinct labeling profiles were obtained for several probes, emphasizing that not only the substitution pattern of the heterocycle but also the heteroatom and its corresponding reactivity contribute significantly to differential proteomic binding preferences.

In order to reveal the identity of these enzymes, a quantitative proteome analysis was performed by adding a trifunctional rhodamine-biotin azide tag under CC conditions for the visualization, enrichment and identification of proteins by SDS-gel electrophoresis and mass spectrometry, as illustrated in Figure 1. Among the identified target enzymes there are several drug target candidates which are essential for bacterial viability, such as MurA2, FabH, FabF and LplA. The results for ThiOc and ThiBenz in *S. aureus* as well as those for ThiOc and OxyOc in *L. welshimeri* are shown in Figures 2 and 3, respectively.

FabH and FabF represent two enzymes with central roles in primary metabolism that are essential for type II fatty acid synthesis and thus presumably for bacterial survival (Wang, J. et al., Proc Natl Acad Sci USA, 2007, 104(18): 7612-7616; Wang, J. et al., Nature, 2006, 441(7091): 358-361). Interestingly, while several oxirane based probes were capable of labeling only FabH in *S. aureus* and *L. welshimeri*, thiirane compounds according to the invention, including the probes ThiOc and ThiBenz, accomplished the labeling of both FabH and FabF in *S. aureus* and *L. welshimeri.*

FabH is the initiation condensing enzyme and FabF the elongation condensing enzyme of fatty acid biosynthesis which are highly conserved among key pathogens. Both enzymes form a covalent bond with the catalytic cysteine in the active site and accommodate the fatty acid substrate in a hydrophobic pocket, explaining the preferred labeling by the octyl-decorated probe (ThiOc) and further emphasizing that not only reactivity but also affinity is an important feature of the three-membered heterocycle based probes.

DAPE (diaminopimelate epimerase) belongs to the family of isomerases, specifically those racemases and epimerases acting on amino acids and derivatives. This enzyme participates in lysine biosynthesis and is thought to be a druggable antibacterial target. (Born, T. L., Blanchard, J., S., Current Opinion in Chemical Biology, Volume 3, Issue 5, , Pages 607-613.)

SufC is involved in the biosynthesis of iron-sulfur clusters (Fe-S). The SUF system of a variety of bacteria is important for Fe-S biogenesis under stressful conditions. The SUF system is made of six proteins with SufC being one. It is described as an atypical cytoplasmic ABC-ATPase, which forms a functional complex with SufB and SufD for the biosynthesis of iron-sulfur clusters. (Eccleston, J., F., Petrovic, A., Davis, C., T., Rangachari, K., and Wilson, R. J. M., J. Biol. Chem. 2006 281: 8371-8378.

MGRA has been shown to affect multiple *Staphylococcus* aureusgenes involved in virulence and antibiotic resistance. It contains a DNA-binding helix-turn-helix motif, which acts as a small transcriptional regulator related to the SarA family of regulators. MgrA regulates certain target genes by directly binding their promoter region (Luong, Thanh T., Dunman, Paul M., Murphy, Ellen, Projan, Steven J., Lee, Chia Y. Transcription Profiling of the mgrA Regulon in

Staphylococcus aureus J. Bacteriol. 2006 188: 1899-1910)

Evaluation of the antibiotic effect of compounds according to the invention

As FabH and FabF, which were identified as targets for the thiiranes and Michael acceptor compounds according to the invention, represent key enzymes in the fatty acid biosynthesis pathway (Wang, J. et al., Proc Natl Acad Sci USA, 2007, 104(18): 7612-7616; Wang, J. et al., Nature, 2006, 441(7091): 358-361), the inventors were interested in further evaluating the antibiotic potential of these compounds, including ThiOc, as well as analogous oxirane and aziridine compounds. Previous studies with the antibacterial compound platencin, a dual FabH/FabF inhibitor, already suggested a potent antibiotic effect resulting from the inhibition of both of these enzymes in parallel.

In order to evaluate the antibiotic effect of ThiOc and the further thiirane, oxirane, aziridine and Michael acceptor compounds described above, the minimal inhibitory concentration (MIC) of these compounds as well as the reference compound cerulenin against multidrug-resistant *S. aureus* (MRSA) Mu50 and *L. monocytogenes* EGD-e was determined, as described in the following.

Probe-mediated growth inhibition of *L. monocytogenes* EGD-e and *S. aureus* Mu50 was determined in 96 well plate-based assays. Various concentrations of oxiranes and thiiranes (1 μL dissolved in DMSO) were added to 99 μL of medium, which contained a fresh 1:1000-fold dilution of the corresponding bacterial overnight culture. The well plates were incubated at 37 °C for 14 h under shaking at 260 rpm. The MIC value was determined by several (> 3) independent experiments (each experiment with at least triplicate runs for each concentration) and represented the lowest concentration of probe at which no growth of bacteria could be observed.

The following MIC values were thereby obtained:

| **Compound** | **MIC for *L. monocytogenes*** | **MIC for multidrug-resistant S. *aureus* (MRSA) Mu50** |
|---|---|---|
| AziEt | >500 μm | >500 μM |
| AziOc | >500 μM | >500 μM |
| AziBenz | >500 μM | >500 μM |
| OxyEt | >500 μM | >500 μM |
| OxyOc | >500 μM | >500 μM |
| OxyBenz | >500 μM | >500 μM |
| OxyAll | >500 μM | >500 μM |
| ThiEt | 500 μM | 500 μM |
| ThiBut | 150 μM | 150 μM |
| ThiHex | 100 μM | 100 μM |
| ThiOc | 75 μM | 75 μM |
| ThiDodec | 500 μM | >500 μM |
| ThiBenz | 500 μM | 500 μM |
| ThiAll | 100 μM | 200 μM |
| MichEt | >500 μM | 500 μM |
| MichOc | 200 μM | 100 μM |
| MichBenz | 150 μM | 200 μM |
| MichAll | 150 μM | 500 μM |
| Cerulenin | 150 μM | 200 μM |

As can be seen, potent antibacterial activity was observed for the thiirane and Michael acceptor compounds according to the invention, including particularly ThiOc, against multidrug-resistant *S. aureus* and *L. monocytogenes.* In contrast thereto, the analogous oxirane and aziridine compounds exhibited no antibacterial activity in this assay.

These results correlate well with the above described dual labeling of FabH and FabF by the thiirane compound ThiOc according to the invention but not by the analogous oxirane compound OxyOc, suggesting - without wishing to

be bound by theory - that the binding to these two targets is indeed responsible for the antibiotic activity observed for the compounds of the present invention.

Importantly, the compound ThiOc according to the invention was found to exert potent antibiotic activity against multidrug-resistant *S. aureus* (MRSA), suggesting that the novel mechanism of action of the compounds according to the invention, including ThiOc, can not be skirted by current resistant pathogens. This opens an intriguing perspective to utilize the compounds provided herein, including the thiirane based compounds according to the invention, as novel lead compounds for anti-bacterial therapy that is even effective against currently untreatable MRSA strains.

It has thus been demonstrated that the thiirane and Michael acceptor compounds according to the present invention, including the compounds of formula (I) and particularly the compounds ThiOc and ThiBenz, bind to the bacterial targets FabH and FabF. In accordance therewith, the compounds of the invention have been found to show potent antibiotic activity against pathogenic bacteria such as multidrug-resistant *S. aureus* (MRSA) and *L. monocytogenes*, which indicates their usefulness in the treatment or prevention of bacterial infections, including, e.g., infections with *S, aureus* or *L. monocytogenes* and particularly infections with multidrug-resistant *S. aureus.*

## Claims

1. A compound of formulae (I)

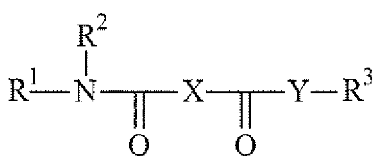

(I)

wherein:
X is

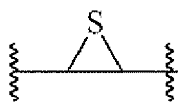 or 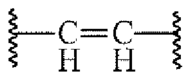;

Y is -O- or -S-;
$R^1$ is selected from $C_{2-8}$ alkynyl, $C_{1-8}$ alkyl, or $C_{2-8}$ alkenyl, wherein said alkynyl, said alkyl or said alkenyl is optionally interrupted by one or more groups independently selected from -O-, -S-, -N(H)-, -N($C_{1-5}$ alkyl)-, -C(=O)-, -C(=O)-N(H)-, -C(=O)-N($C_{1-5}$ alkyl)-, -N(H)-C(=O)-, or -N($C_{1-5}$ alkyl)-C(=O)-, and further wherein said alkynyl, said alkyl or said alkenyl is optionally substituted with one or more groups independently selected from halogen, $-CF_3$, -CN, -OH, -O($C_{1-5}$ alkyl), -SH, -S($C_{1-5}$ alkyl), $-NH_2$, -NH($C_{1-5}$ alkyl), -N($C_{1-5}$ alkyl)($C_{1-5}$ alkyl), -CO-($C_{1-5}$ alkyl), $-CO-NH_2$, -CO-NH-($C_{1-5}$ alkyl), -CO-N($C_{1-5}$ alkyl)($C_{1-5}$ alkyl), -NH-CO-($C_{1-5}$ alkyl), -N($C_{1-5}$ alkyl)-CO-($C_{1-5}$ alkyl), aryl, or heteroaryl;
$R^2$ is H or $C_{1-5}$ alkyl; and
$R^3$ is selected from $C_{1-14}$ alkyl, $C_{2-14}$ alkenyl, or $C_{2-14}$ alkynyl, wherein said alkyl, said alkenyl or said alkynyl is optionally interrupted by one or more groups independently selected from -O-, -S-, -N(H)-, -N($C_{1-5}$ alkyl)-, -C(=O)-, -C(=O)-N(H)-, -C(=O)-N($C_{1-5}$ alkyl)-, -N(H)-C(=O)-, or -N($C_{1-5}$ alkyl)-C(=O)-, and further wherein said alkyl, said alkenyl or said alkynyl is optionally substituted with one or more groups independently selected from halogen, $-CF_3$, -CN, -OH, -O($C_{1-5}$ alkyl), -SH, -S($C_{1-5}$ alkyl), $-NH_2$, -NH($C_{1-5}$ alkyl), -N($C_{1-5}$ alkyl)($C_{1-5}$ alkyl), -CO-($C_{1-5}$ alkyl), $-CO-NH_2$, -CO-NH-($C_{1-5}$ alkyl), -CO-N($C_{1-5}$ alkyl)($C_{1-5}$ alkyl), -NH-CO-($C_{1-5}$ alkyl), or -N($C_{1-5}$ alkyl)-CO-($C_{1-5}$ alkyl), aryl, or heteroaryl;
or a pharmaceutically acceptable salt, solvate or prodrug thereof.

2. The compound of claim 1, wherein X is

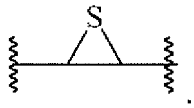

3. The compound of claim 1, wherein X is

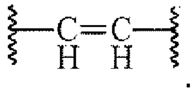

4. The compound of any of claims 1 to 3, wherein Y is -O-.

5. The compound of any of claims 1 to 4, wherein $R^1$ is selected from $C_{1-8}$ alkyl, $C_{2-8}$ alkenyl, or $C_{2-8}$ alkynyl, wherein said alkyl, said alkenyl or said alkynyl is optionally interrupted by one or more -O- groups, and further wherein said alkyl, said alkenyl or said alkynyl is optionally substituted with one or more groups independently selected from halogen, $-CF_3$, -CN, -OH, -O($C_{1-5}$ alkyl), -SH, -S($C_{1-5}$ alkyl), $-NH_2$, -NH($C_{1-5}$ alkyl), or -N($C_{1-5}$ alkyl)($C_{1-5}$ alkyl).

6. The compound of any of claims 1 to 5, wherein $R^3$ is selected from $C_{1-10}$ alkyl, $C_{2-10}$ alkenyl, or $C_{2-10}$ alkynyl, wherein said alkyl, said alkenyl or said alkynyl is optionally substituted with one group selected from halogen, $-CF_3$, -CN, -OH, -O($C_{1-5}$ alkyl), -SH, -S($C_{1-5}$ alkyl), $-NH_2$, -NH($C_{1-5}$ alkyl), -N($C_{1-5}$ alkyl)($C_{1-5}$ alkyl), or phenyl.

7. The compound of claim 1, wherein the compound is a compound selected from:

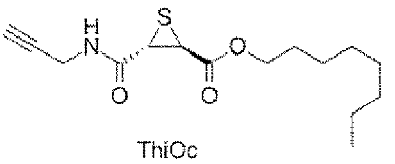

ThiOc

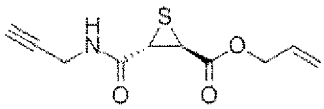

ThiAll

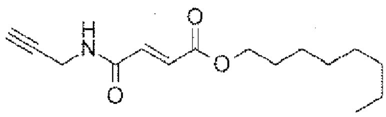

MichOc

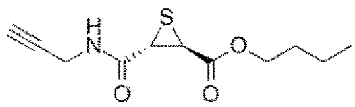

ThiBut

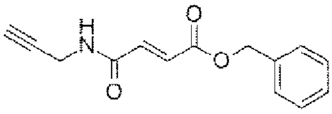

MichBenz

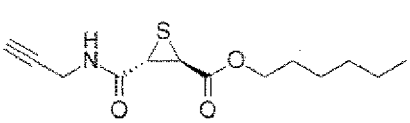

ThiHex

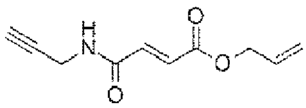

MichAll

or a pharmaceutically acceptable salt, solvate or prodrug thereof.

8. A pharmaceutical composition comprising the compound of any of claims 1 to 7 and optionally a pharmaceutically acceptable excipient.

**9.** The compound of any of claims 1 to 7 or the pharmaceutical composition of claim 8 for use in treating or preventing a bacterial infection.

**10.** The compound of claim 9 or the pharmaceutical composition of claim 9, wherein the bacterial infection is caused by Gram-positive bacteria.

**11.** The compound of claim 9 or 10 or the pharmaceutical composition of claim 9 or 10, wherein the bacterial infection is caused by *Listeria monocytogenes*, *Listeria ivanovii*, *Staphylococcus aureus, Staphylococcus epidermidis, Staphylococcus saprophyticus, Staphylococcus lugdunensis, Staphylococcus schleiferi, Staphylococcus caprae, Streptococcus pneumoniae, Streptococcus viridans, Streptococcus pyogenes, Streptococcus agalactiae, Enterococcus faecalis, Enterococcus faecium, Bacillus licheniformis*, *Bacillus subtilis, Bacillus anthracis*, *Bacillus cereus, Bacillus thuringiensis, Bacillus larvae, Mycobacterium tuberculosis, Mycobacterium leprae, Mycobacterium ulcerans, Mycobacterium kanasasii, Mycobacterium avium, Mycobacterium paratuberculosis, Mycobacterium scrofulaceam, Mycobacterium microti, Mycobacterium africanum, Mycobacterium canettii, Mycobacterium intracellulare, Mycobacterium simiae, Mycobacterium szulgai, Mycobacterium xenopi, Mycobacterium fortuitum, Mycobacterium chelonei*, *Mycobacterium marinum, Nocardia asteroides*, or *Rhodococcus equi.*

**12.** The compound of any of claims 9 to 11 or the pharmaceutical composition of any of claims 9 to 11, wherein the bacterial infection is caused by multidrug-resistant *Staphylococcus aureus.*

**13.** The compound of claim 9 or the pharmaceutical composition of claim 9, wherein the bacterial infection is caused by Gram-negative bacteria.

**14.** The compound of claim 13 or the pharmaceutical composition of claim 13, wherein the bacterial infection is caused by *Neisseria gonorrhoeae*, *Neisseria meningitidis*, *Moraxella catarrhalis, Haemophilus influenzae, Klebsiella pneumoniae, Legionella pneumophila, Pseudomonas aeruginosa, Pseudomonas putida, Escherichia coli, Proteus mirabilis*, *Enterobacter cloaceae, Serratia marcescens, Helicobacter pylori, Salmonella enteritidis*, or *Salmonella typhi.*

**15.** The compound of claims 9 or the pharmaceutical composition of claim 9, wherein the bacterial infection is selected from listeriosis, cranial nerve palsy, encephalitis, meningitis, meningoencephalitis, abscesses, endocarditis, pneumonia, cholera, syphilis, diphtheria, anthrax, leprosy, tuberculosis, bubonic plague, sepsis, septic arthritis, osteitis, inflammation of wounds, furuncles, carbuncles, toxic shock syndrome, Staphylococcal scalded skin syndrome, or mastitis.

Fig. 1

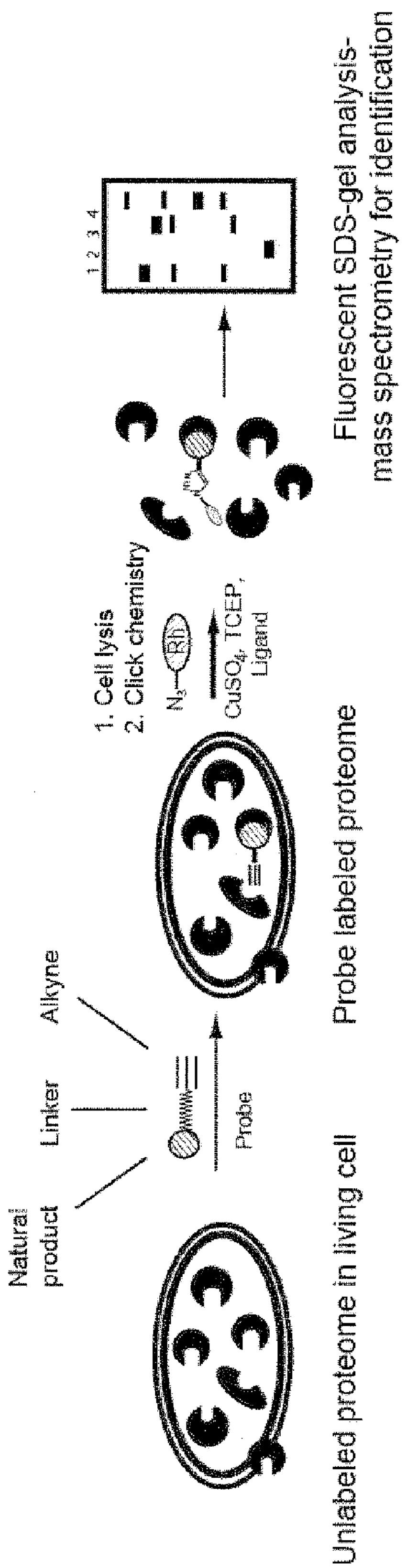
Natural product
Linker
Alkyne
Probe
Unlabeled proteome in living cell
Probe labeled proteome
1. Cell lysis
2. Click chemistry
$N_3$—Rh
$CuSO_4$, TCEP, Ligand
1 2 3 4
Fluorescent SDS-gel analysis-mass spectrometry for identification

Fig. 2

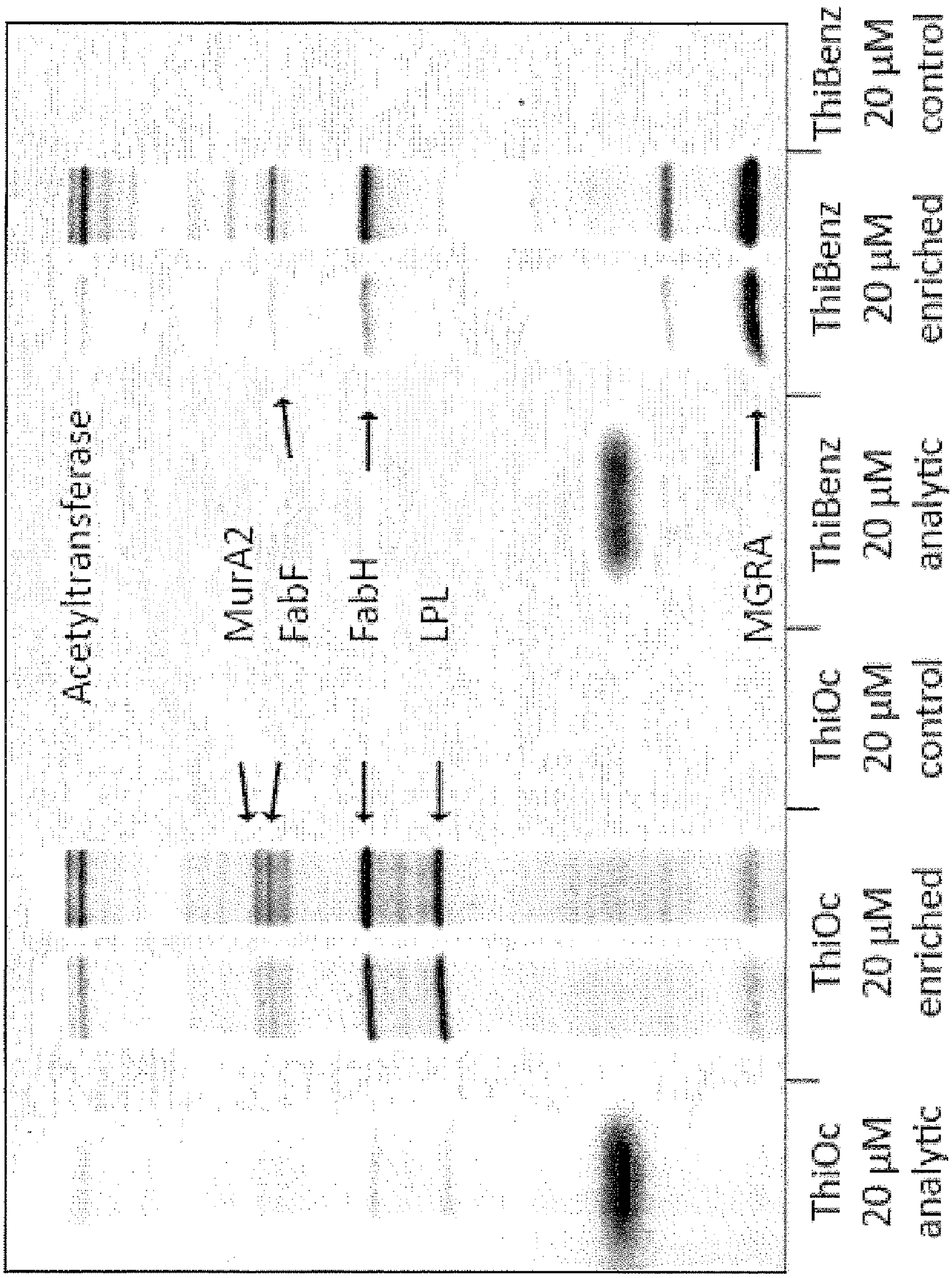
Acetyltransferase
MurA2
FabF
FabH
LPL
MGRA
ThiOc 20 µM analytic
ThiOc 20 µM enriched
ThiOc 20 µM control
ThiBenz 20 µM analytic
ThiBenz 20 µM enriched
ThiBenz 20 µM control

Fig. 3

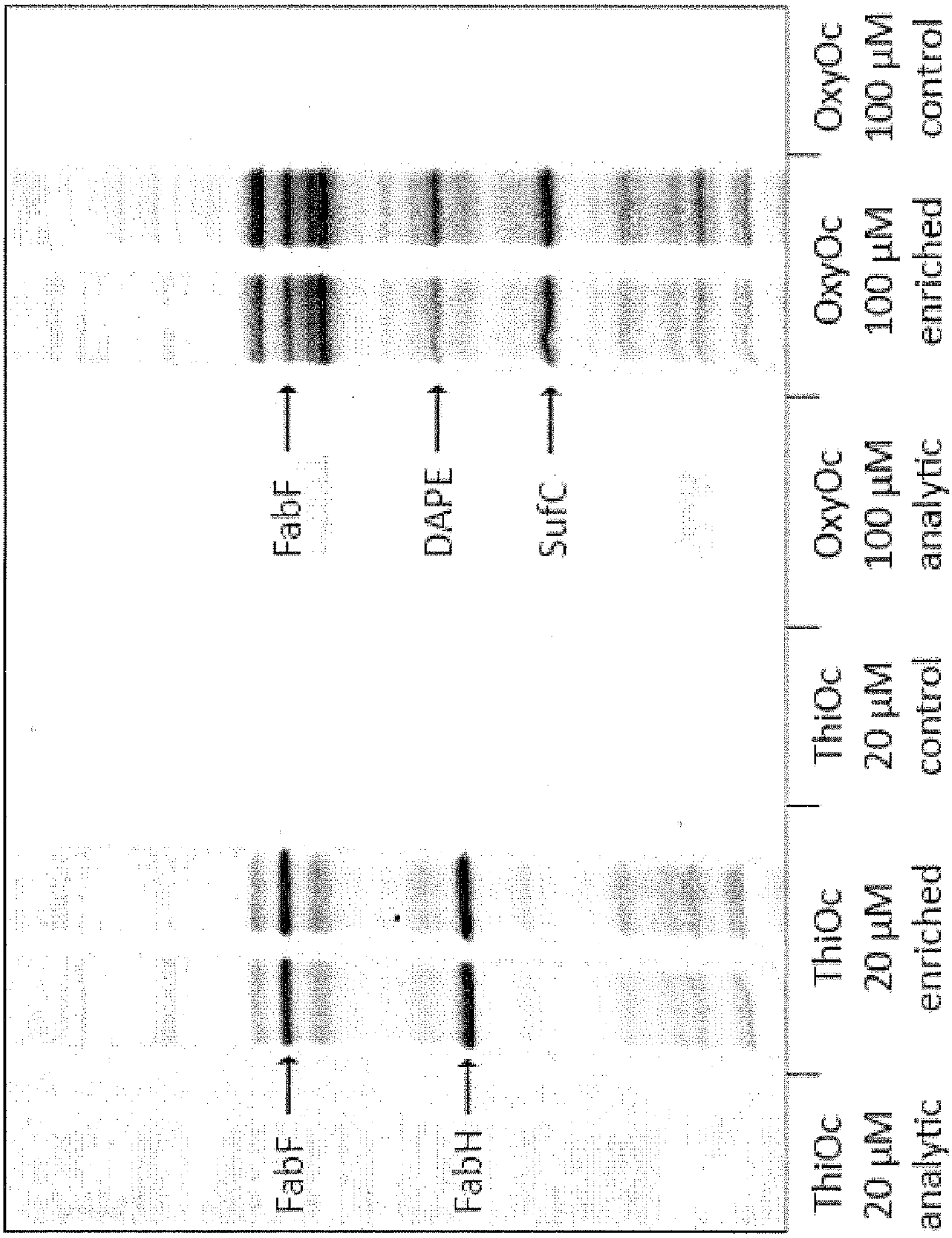
FabF
FabH
FabF
DAPE
SufC
ThiOc 20 µM analytic
ThiOc 20 µM enriched
ThiOc 20 µM control
OxyOc 100 µM analytic
OxyOc 100 µM enriched
OxyOc 100 µM control

Europäisches Patentamt
European Patent Office
Office européen des brevets

# EUROPEAN SEARCH REPORT

Application Number
EP 11 19 4584

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WEI REN ET AL: "Carbamoylation of Aryl Halides by Molybdenum or Tungsten Carbonyl Amine Complexes", THE JOURNAL OF ORGANIC CHEMISTRY, vol. 75, no. 9, 7 May 2010 (2010-05-07), pages 3017-3020, XP55022542, ISSN: 0022-3263, DOI: 10.1021/jo1002592 * page 3019; table 2; compound 3n * ----- | 1,3-6 | INV. C07D331/02 A61P31/04 |
| X | ALBERT SÁNCHEZ ET AL: "Esterification of Maleamic Acids without Double Bond Isomerization", EUROPEAN JOURNAL OF ORGANIC CHEMISTRY, vol. 2010, no. 13, 1 May 2010 (2010-05-01), pages 2600-2606, XP55022547, ISSN: 1434-193X, DOI: 10.1002/ejoc.200901365 * page 2601; table 1; compound 2; sequence 2 * * page 2602; table 2 * * page 2603; compounds 2,5,7,12,13,16,17,19 * ----- | 1,3-6 | |
| | | | TECHNICAL FIELDS SEARCHED (IPC) |
| X | US 2005/124598 A1 (EATON BRUCE [US] ET AL) 9 June 2005 (2005-06-09) * page 4 * ----- | 1,3-6 | C07D |
| X | SHORT K M ET AL: "An addition-elimination strategy for the synthesis of oxazoles", TETRAHEDRON LETTERS, ELSEVIER, AMSTERDAM, NL, vol. 34, no. 1, 1 January 1993 (1993-01-01), pages 71-74, XP026637171, ISSN: 0040-4039, DOI: 10.1016/S0040-4039(00)60060-6 [retrieved on 1993-01-01] * page 72; table 1; compound e * ----- -/-- | 1,3-6 | |

The present search report has been drawn up for all claims

1

| Place of search | Date of completion of the search | Examiner |
|---|---|---|
| The Hague | 28 March 2012 | Megido, Benigno |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 19 4584

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALESSIA BACCHI ET AL: "Efficient and General Synthesis of 5-(Alkoxycarbonyl)methylene-3-oxazolines by Palladium-Catalyzed Oxidative Carbonylation of Prop-2-ynylamides", THE JOURNAL OF ORGANIC CHEMISTRY, vol. 67, no. 13, 1 June 2002 (2002-06-01), pages 4450-4457, XP55022551, ISSN: 0022-3263, DOI: 10.1021/jo0200217 * page 4453; compound 1f; sequence 5 * ----- | 1,3-6 | |
| X | MORIMOTO ET AL: "Effect of a thiol proteinase inhibitor, E-64-d, on susceptibility to infection with Staphylococcus aureus in Chediak-Higashi syndrome (beige) mice", INTERNATIONAL IMMUNOPHARMACOLOGY, ELSEVIER, AMSTERDAM, NL, vol. 7, no. 7, 10 May 2007 (2007-05-10), pages 973-980, XP022070038, ISSN: 1567-5769, DOI: 10.1016/J.INTIMP.2007.03.004 * pages 974,978; compounds E-64-d * ----- | 1-15 | TECHNICAL FIELDS SEARCHED (IPC) |
| X | SILVIA REGINA TOZATO PRADO ET AL: "Biological Evaluation of Some Selected Cyclic Imides: Mitochondrial Effects and in vitro Cytotoxicity", ZEITSCHRIFT FUER NATURFORSCHUNG. C, A JOURNAL OF BIOSCIENCES, TUEBINGEN, DE, vol. 59C, 1 January 2004 (2004-01-01), pages 663-672, XP007920422, ISSN: 0939-5075 * pages 664,668; compound S3 * ----- | 1-15 | |

The present search report has been drawn up for all claims

| Place of search | Date of completion of the search | Examiner |
|---|---|---|
| The Hague | 28 March 2012 | Megido, Benigno |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 19 4584

This annex lists the patent family members relating to the patent documents cited in the above-mentioned European search report.
The members are as contained in the European Patent Office EDP file on
The European Patent Office is in no way liable for these particulars which are merely given for the purpose of information.

28-03-2012

| Patent document cited in search report | Publication date | Patent family member(s) | Publication date |
| --- | --- | --- | --- |
| US 2005124598 A1 | 09-06-2005 | AU 2002359761 A1 | 30-06-2003 |
| | | US 2005124598 A1 | 09-06-2005 |
| | | WO 03051314 A2 | 26-06-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5539132 A

### Non-patent literature cited in the description


- **TAUBES, G.** *Science,* 2008, vol. 321 (5887), 356-361
- **CLARDY, J. et al.** *Nat Biotechnol,* 2006, vol. 24 (12), 1541-1550
- **CLARDY et al.** *Nature,* 2004, vol. 432 (7019), 829-837
- **OMURA S.** *Bacteriol Rev,* 1976, vol. 40 (3), 681-697
- **YOUNG K. et al.** *Antimicrob Agents Chemother,* 2006, vol. 50 (2), 519-526
- **CHEW, W.A.H.D.** *Sulfur reports,* 1993, vol. 15, 1-39
- **WANG, J. et al.** *Proc Natl Acad Sci USA,* 2007, vol. 104 (18), 7612-7616
- **WANG, J. et al.** *Nature,* 2006, vol. 441 (7091), 358-361
- **PRICE, A.C. et al.** *J Biol Chem.,* 2001, vol. 276 (9), 6551-6559
- **MORI, K. ; IWASAWA, H.** *Tetrahedron,* 1980, 87-90
- **MEARA, J. P. ; RICH, D. H.** *J Med Chem,* 1996, vol. 39 (6), 3357-3366
- **KORN, A. C. ; MORODER L.** *J Chem Res (S,* 1995, 102-103
- **BUNDGAARD, H.** Design of Prodrugs. Elsevier, 1985, 7-921-24
- Remington's Pharmaceutical Sciences
- **SIEBER, S.A. et al.** *Nat Chem Biol,* 2006, vol. 2 (5), 274-281
- **EVANS, M. J. ; CRAVATT, B. F.** *Chem Rev,* 2006, vol. 106, 3279
- **CRAVATT, B. F. ; WRIGHT, A. T. ; KOZARICH, J. W.** *Annu. Rev. Biochem.,* 2008, vol. 77, 383
- **BORN, T. L. ; BLANCHARD, J., S.** *Current Opinion in Chemical Biology,* vol. 3 (5), 607-613
- **ECCLESTON, J., F. ; PETROVIC, A. ; DAVIS, C., T. ; RANGACHARI, K. ; WILSON, R. J. M.** *J. Biol. Chem.,* 2006, vol. 281, 8371-8378
- **LUONG, THANH T. ; DUNMAN, PAUL M. ; MURPHY, ELLEN, PROJAN, STEVEN J. ; LEE, CHIA Y.** Transcription Profiling of the mgrA Regulon in Staphylococcus aureus. *J. Bacteriol.,* 2006, vol. 188, 1899-1910